# EUROPÄISCHE PATENTSCHRIFT

(11) **EP 3 795 082 B1**
(45) Veröffentlichungstag und Bekanntmachung des Hinweises auf die Patenterteilung: **29.06.2022**
(21) Anmeldenummer: 19198986.2
(22) Anmeldetag: 23.09.2019
(51) Int. Cl.: A61B 6/00, A61B 6/03

(54) **VERFAHREN UND VORRICHTUNG ZUR ERZEUGUNG EINES SPEKTRALEN COMPUTERTOMOGRAPHIE-BILDDATENSATZES**
METHOD AND APPARATUS FOR GENERATING A SPECTRAL COMPUTER TOMOGRAPHY IMAGE DATA SET
PROCÉDÉ ET DISPOSITIF DE GÉNÉRATION D'UN ENSEMBLE DE DONNÉES D'IMAGE DE TOMODENSITOMÉTRIE

(43) Veröffentlichungstag der Anmeldung: 24.03.2021
(73) Patentinhaber: Siemens Healthcare GmbH, 91052 Erlangen (DE)
(72) Erfinder: Flohr, Thomas, 91486 Uehlfeld (DE); Schmidt, Bernhard, 90766 Fürth (DE)

(56) Entgegenhaltungen:
- DE-A1-102007 024 158
- US-A1- 2005 082 491
- US-B2- 8 155 422

## Beschreibung

Die Erfindung betrifft ein Verfahren, eine Vorrichtung, ein Computertomographiesystem und ein Computerprogramm zur Erzeugung eines spektralen Computertomographie-Röntgenbilddatensatzes.

Die Computertomographie (CT) ist ein bildgebendes Verfahren, welches vor allem zur medizinischen Diagnostik eingesetzt wird. Bei der CT rotieren zur Aufnahme räumlich dreidimensionaler Bilddaten eine Strahlungsquelle, beispielsweise eine Röntgenquelle, sowie ein mit dieser zusammenwirkender Röntgendetektor um ein zu untersuchendes Untersuchungsobjekt, insbesondere einen Patienten. Während der Rotationsbewegung werden jeweils innerhalb eines Winkelsektors Messdaten, sogenannte Projektionsmessdaten aufgenommen. Bei den Projektionsmessdaten handelt es sich um eine Projektion oder eine Mehrzahl von Projektionen, welche Informationen über die Schwächung der Strahlung durch das Untersuchungsobjekt aus verschiedenen Projektionswinkeln enthalten. Aus diesen Projektionsmess-daten, auch Rohdatensätze genannt, lässt sich ein zweidimensionales Schnittbild oder ein dreidimensionales Volumenbild des Untersuchungsobjektes, beispielsweise mittels der sogenannten gefilterten Rückprojektion oder mittels eines iterativen Rekonstruktionsverfahrens, rekonstruieren.

Die Messdaten enthalten auch spektrale Information, da die Absorption im Untersuchungsobjekt abhängig von der Energie der Röntgenstrahlung ist, d.h. der Energie der Röntgenphotonen, welche das Untersuchungsobjekt passiert. Diese spektrale Information kann Hinweise auf die Zusammensetzung des Untersuchungsobjekts liefern und eine Unterscheidbarkeit zwischen unterschiedlichen Materialien im Untersuchungsobjekt ermöglichen. Diese sind jedoch mittels eines konventionellen CT-Geräts mit einem energieintegrierenden Röntgendetektor üblicherweise nicht zugänglich.

Eine Möglichkeit Zugang zu der spektralen Informationen zu erhalten liegt in der Aufnahme von Projektionsmessdaten mit Röntgenstrahlung mit mindestens zwei, voneinander verschiedenen Energiespektren, beispielsweise einem niederenergetischen und einem höherenergetischem Energiespektrum, und der gemeinsamen Verarbeitung der daraus resultierenden Messdaten mittels einer spektralen Datenverarbeitungstechnik. Hier ist der Einsatz sogenannter Dual Source CT-Geräte bekannt ("Zwei Quellen" CT-Geräte), welche zwei winkelversetzt angeordnete Röntgenquellen mit jeweils gegenüberliegenden Röntgendetektoren aufweisen, und wobei die zwei Röntgenquellen zwei voneinander verschiedene Energiespektren emittieren. Auch ist der Einsatz einer Röntgenröhre mit einem sogenannten Split-Filter, d.h. mit einem z.B. entlang der Rotationsachse des CT-Geräts geteilten Vorfilter, bekannt, wobei in unterschiedliche Raumbereiche gleichzeitig zwei voneinander verschiedene Energiespektren emittiert werden und damit unterschiedliche Bereiche eines Röntgendetektors mit unterschiedlichen Energiespektren belichtet werden. Darüber hinaus ist auch die Möglichkeit des sogenannten kV-Switchings ("kV-Umschaltens") zu erwähnen, wobei eine Röntgenquelle eingesetzt wird, welche ausgebildet ist, abwechselnd in schneller zeitlicher Abfolge Röntgenstrahlung mit zwei unterschiedlichen Energiespektren zu emittieren, indem abwechselnd in schneller zeitlicher Abfolge zwischen zwei Röhrenspannung umgeschaltet wird.

Neben dem Einsatz von Röntgenstrahlung mit zumindest zwei voneinander verschiedenen Energiespektren, können spektrale CT-Untersuchungen auch mittels des Einsatzes energieauflösender Röntgendetektoren ermöglicht werden. Beispielsweise kann ein photonenzählenden, direkt-konvertierende Röntgendetektoren eingesetzt werden. Eintreffende Röntgenstrahlung bzw. Photonen können in solchen Röntgendetektoren durch ein geeignetes Konvertermaterial in elektrische Pulse umgewandelt werden. Als Konvertermaterial können beispielsweise CdTe, CZT, HgI2, GaAs oder andere verwendet werden. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung dann durch Zählen der elektrischen Pulse, welche durch die Absorption von Röntgenphotonen im Konvertermaterial ausgelöst werden, gemessen. Die Höhe oder auch die Länge eines erzeugten elektrischen Pulses ist in der Regel proportional zur Energie des absorbierten Röntgenphotons. Dadurch kann eine spektrale Information durch den Vergleich der Höhe bzw. Länge des elektrischen Pulses mit einer Energieschwelle extrahiert werden. Werden beispielsweise zwei Energieschwellen für einen Vergleich bereitgestellt, können Messsignale generiert bzw. ausgegeben werden, welche in zwei, durch die zwei Energieschwellen definierte Energiebereiche, auch Energietöpfe genannt, aufgelöst sind. Darüber hinaus sind auch sogenannte Dual-Layer ("Zweischicht"-) Röntgendetektoren bekannt, welche den Zugang zu spektraler Information ermöglichen können.

In spektralen CT-Untersuchungen ist ein wesentliches Kriterium für die Qualität von spektralen Rohdaten- oder Bildverarbeitungsalgorithmen (z. B. zur Berechnung von Basismaterialbildern, zur Berechnung von pseudo-monoenergetischen Bildern, oder zur Klassifizierung von Materialien im CT-Bild) die spektrale Trennung der mindestens zwei spektral unterschiedlichen Rohdatensätze bzw. Messdatensätze, die für die spektrale Datenverarbeitungstechnik verwendet werden. Idealerweise würde man mit im Röntgenenergiebereich völlig getrennten spektralen Eingangsdatensätzen arbeiten, was sich jedoch mit den bekannten Verfahren nicht erreichen lässt.

Die Druckschrift US 2005/082491 A1 offenbart die Aufnahme von zwei Bilddatensätzen insbesondere unter Nutzung von Strahlung mit unterschiedlichen Energielevel, wobei die Detektionseffizient der Detektorvorrichtung für ein erstes Energielevel durch Nutzen eines ersten Filters und ein zweites Energielevel durch Nutzen eines zweiten Filters optimiert werden kann. Dabei wird außerdem offenbart, dass die Detektorvorrichtung ausgebildet sein kann, eine Photonenpulsamplitude und/oder einen Photonenanzahl zu detektieren.

Die Druckschrift US 8 155 422 B2 offenbart eine Methode für eine dynamische Optimierung des Signal-zu-Rausch Verhältnisses von Abschwächungsdaten, welche mit zwei unterschiedlichen Röntgenenergien verbunden sind, für eine Rekonstruktion von Bilddaten eines Objekts.

Die Druckschrift DE 10 2007 024158 A1 offenbart ein Computertomographie-Gerät mit zwei Röntgenquellen, welche mit zwei verschiedenen Röhrenspannungen betrieben werden können.

Aufgabe der Erfindung ist es daher ein verbessertes Verfahren zur Erzeugung eines spektralen CT-Bilddatensatz bereitzustellen, wobei eine spektrale Trennung der Messsignale verbessert ermöglicht wird.

Die Aufgabe wird gelöst durch die Merkmale der unabhängigen Patentansprüche. Weitere vorteilhafte und teils für sich erfinderische Ausführungsformen und Weiterbildungen der Erfindung sind in den Unteransprüchen und der nachfolgenden Beschreibung dargelegt.

Nachstehend wird die erfindungsgemäße Lösung der Aufgabe sowohl in Bezug auf das beanspruchte Verfahren als auch in Bezug auf die beanspruchte Vorrichtung beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung betrifft ein Verfahren zur Erzeugung eines spektralen Computertomographie-Bilddatensatzes (CT-Bilddatensatz) mittels einer Detektionseinheit, aufweisend zumindest einen photonenzählenden Röntgendetektor, und einer Röntgenquelleneinheit umfassend die Schritte des Anpassens, des Emittierens, des Detektierens, des Erzeugens und des Ausgebens.

Die Röntgenquelleneinheit ist erfindungsgemäß dazu ausgebildet, Röntgenstrahlung aufweisend ein erstes Energiespektrum und aufweisend ein zweites, vom ersten verschiedenes Energiespektrum zu emittieren. Die Röntgenquelleneinheit umfasst dafür zumindest eine Röntgenquelle. Die Röntgenquelle kann beispielsweise als Röntgenröhre ausgebildet sein. Die Röntgenquelleneinheit kann jedoch auch mehr als eine Röntgenquelle umfassen. Im Falle eine Dual-Source CT-Geräts umfasst die Röntgenquelleneinheit beispielsweise zwei winkelversetzt angeordnete Röntgenquellen, welche um eine gemeinsame Rotationsachse um das Untersuchungsobjekt für die Aufnahme von Messsignalen innerhalb unterschiedlicher Winkelsektoren rotierbar angeordnet sind.

Die Röntgenquelleneinheit kann ausgebildet sein das erste und das zweite Energiespektrum zu emittieren, in dem die zumindest eine Röntgenquelle ausgebildet ist, gleichzeitig oder zeitlich versetzt sowohl das erste als auch das zweite Energiespektrum zu emittieren. Die Röntgenquelleneinheit kann beispielsweise eine Röntgenquelle aufweisen, welche ausgebildet ist, das erste und das zweite Energiespektrum gleichzeitig in zwei unterschiedliche Raumbereiche, etwa mittels eines Split-Filters, zu emittieren. Die Röntgenquelle kann zu einer Methode des kV-Switchings ausgebildet sein und zeitlich aufeinanderfolgend entweder das erste oder das zweite Energiespektrum emittieren. Die Röntgenquelleneinheit kann auch ausgebildet sein das erste und das zweite Energiespektrum zu emittieren, indem die Röntgenquelleneinheit zwei Röntgenquellen umfasst, wobei die erste der zwei Röntgenquellen, beispielsweise das erste Energiespektrum, und die zweite der zwei Röntgenquellen das zweite Energiespektrum emittiert.

Ein Energiespektrum emittierter Röntgenstrahlung beschreibt im Wesentlichen die Verteilung der emittierten Röntgenphotonen in Abhängigkeit der Photonenenergie. Das erste emittierte Energiespektrum kann sich zumindest dadurch von dem zweiten Energiespektrum unterscheiden, dass sich die mittlere, emittierte Energie der Röntgenstrahlung aufweisend das erste Energiespektrum von der mittleren, emittierten Energie der Röntgenstrahlung aufweisend das zweite Energiespektrum unterscheidet. Das erste und das zweite Energiespektrum kann sich beispielsweise auch dadurch unterscheiden, dass sich die maximale emittierte Energie unterscheidet. Das erste Energiespektrum kann sich auch dadurch vom zweiten Energiespektrum unterscheiden, dass sich die minimal auftretende Energie, welche einen wesentlichen Beitrag zur Erzeugung des CT-Bilddatensatzes leistet, unterscheidet. Die minimal auftretende Energie kann beispielsweise durch einen starken Anstieg der Photonenzahl am niederenergetischen Ende des Energiespektrums gekennzeichnet sein. Zwei unterschiedliche Energiespektren können bevorzugt beispielsweise durch die Verwendung zweier, unterschiedlicher Röhrenspannungen erzeugt werden. Auch der Einsatz unterschiedlicher Vorfilterungen, welche die Röntgenstrahlung zumindest zum Teil absorbiert, oder die Verwendung von unterschiedlichen Anodenmaterialien einer Röntgenröhre führt zu unterschiedlichen Energiespektren.

Die erfindungsgemäße Detektionseinheit ist ausgebildet detektierte Röntgenstrahlung in energieaufgelöste Messsignale umzuwandeln, welche zumindest in einen ersten, anpassbaren Energiebereich und einen zweiten, anpassbaren Energiebereich aufgelöst sind. Die Detektionseinheit weist dazu erfindungsgemäß zumindest einen photonenzählenden Röntgendetektor auf. Der zumindest eine Röntgendetektor umfasst dabei in der Regel eine Mehrzahl an Detektionselementen, auch Pixel genannt, in einer zeilen- oder matrixförmigen Anordnung. Der zumindest eine Röntgendetektor kann dabei aus mehreren Detektormodulen zusammengesetzt sein, jeweils aufweisend eine Teilzahl der Mehrzahl an Detektionselementen des Röntgendetektors. Der zumindest eine Röntgendetektor kann dabei als Bauelement aufgefasst werden, welches einer Röntgenquelle der Röntgenquelleneinheit zugeordnet ist und dieser gegenüberliegend angeordnet ist und von dieser für die Aufnahme von Messdaten, d.h. der Messsignale, belichtet wird. Zwischen dem zumindest einen Röntgendetektor der Detektionseinheit und der zugeordneten Röntgenquelle der Röntgenquelleneinheit ist dabei das Untersuchungsobjekt positioniert.

Die Detektionseinheit kann insbesondere dazu ausgebildet sein detektierte Röntgenstrahlung in Messsignale umzuwandeln, welche zumindest in einen ersten, anpassbaren Energiebereich und einen zweiten, anpassbaren Energiebereich aufgelöst sind, indem der zumindest eine photonenzählende Röntgendetektor dazu ausgebildet ist, detektierte Röntgenstrahlung in energieaufgelöste Messsignale umzuwandeln und in Abhängigkeit von dem ersten und dem zweiten anpassbaren Energiebereich für eine Weiterverarbeitung bereitzustellen. Ebenso ist vorstellbar, dass die Detektionseinheit zwei photonenzählende Röntgendetektoren aufweist, wobei der erfindungsgemäße erste Energiebereich zumindest von einem ersten photonenzählenden Röntgendetektor der zwei Röntgendetektoren und der erfindungsgemäße zweite Energiebereich zumindest von dem zweiten photonenzählenden Röntgendetektor der zwei Röntgendetektoren bereitgestellt sein kann. Es kann jedoch auch andere Konfigurationen geben. Beispielsweise können sowohl der erste als auch der zweite photonenzählende Röntgendetektor Messsignale in Abhängigkeit sowohl von dem ersten als auch dem zweiten Energiebereich bereitstellen.

Der oder die photonenzählenden Röntgendetektoren kann bzw. können darüber hinaus auch ausgebildet sein detektierte Röntgenstrahlung in mehr als zwei Energiebereiche aufzulösen. Beispielsweise können drei, vier oder sechs Energiebereiche bereitgestellt werden. Ebenso kann vorgesehen sein, dass die Energiebereiche für eine Anzahl an Detektionselementen eines photonenzählenden Röntgendetektors, beispielsweise pixelindividuell, anpassbar sind, so dass innerhalb der Matrix an Detektionselementen eines photonenzählenden Röntgendetektors die gleichen aber auch lokal unterschiedliche Energiebereiche für die Erzeugung von Messsignalen bereitgestellt sein können.

Der oder die photonenzählenden Röntgendetektoren können insbesondere ausgebildet sein energieaufgelöste Messsignale in Abhängigkeit von anpassbaren Energiebereichen für eine Weiterverarbeitung bereitzustellen, indem anpassbare Energieschwellen für einen Vergleich der Höhe oder Länge der durch die eintreffende Röntgenstrahlung erzeugten elektrischen Pulse im Röntgendetektor bereitgestellt sind. Insbesondere kann eine untere und/oder obere Grenzenergie eines jeweiligen Energiebereichs mittels einer einstellbaren Energieschwellen anpassbar sein.

In der folgenden Beschreibung wird für eine bessere Verständlichkeit angenommen, dass das erste Energiespektrum zumindest eine niederenergetischere mittlere Energie aufweist als das zweite Energiespektrum. Außerdem wird im Folgenden angenommen, dass der erste Energiebereich einen im Vergleich zum zweiten erfindungsgemäßen Energiebereich niederenergetischeren Bereich an Photonenenergien der Röntgenstrahlung und der zweite erfindungsgemäße Energiebereich einen höherenergetischen Energiebereich abdeckt. Dies soll jedoch eine umgekehrte Konfiguration nicht ausschließen. In umgekehrten Ausführungen, in welchen das erste Energiespektrum eine höhere mittlere Energie aufweist als das zweite Energiespektrum, wären in der folgenden Beschreibung auch die von dem ersten und dem zweiten Energiebereich abgedeckten Bereiche an Photonenenergien umgekehrt zuzuordnen und der Beschreibungstext entsprechend gedanklich anzupassen.

Im erfindungsgemäßen Schritt des Anpassens wird der erste Energiebereich und der zweite Energiebereich in Abhängigkeit des ersten Energiespektrums und des zweiten Energiespektrums mittels einer Anpassungseinheit angepasst, wobei jeweils zumindest eine Grenzenergie eines jeweiligen Energiebereichs angepasst wird. Das Anpassen in Abhängigkeit des ersten Energiespektrums und des zweiten Energiespektrums kann umfassen, dass die jeweilige zumindest eine Grenzenergie in Abhängigkeit des ersten und des zweiten Energiespektrums gewählt und angewendet wird. Daraus kann resultieren, dass die Verwendung von anderen Energiespektren insbesondere zu einer anderen zumindest einen Grenzenergie und damit zu anderen Energiebereichen führt.

Dabei kann zumindest eine erste, obere Grenzenergie des niederenergetischeren, ersten Energiebereichs angepasst werden, welche insbesondere der maximalen, vom ersten Energiebereich umfassten Photonenenergie entspricht. Es kann zumindest eine zweite, untere Grenzenergie des zweiten, höherenergetischen Energiebereichs angepasst werden, welche insbesondere der minimalen, vom zweiten Energiebereich umfassten Photonenenergie entspricht. Darüber hinaus kann auch vorgesehen sein weitere Grenzenergien, beispielsweise eine untere Grenzenergie des niederenergetischeren, ersten Energiebereichs oder eine obere Grenzenergie des zweiten, höherenergetischen Energiebereichs oder Grenzenergien weiterer bereitgestellter Energiebereiche, anzupassen.

In Ausführungsvarianten kann die erste, oberer Grenzenergie des ersten Energiebereichs auf den gleichen Energiewert wie die zweite, untere Grenzenergie des zweiten Energiebereichs angepasst werden. Insofern der erste und der zweite Energiebereich von einem photonenzählenden Röntgendetektor bereitgestellt wird, kann dann die erste, oberer Grenzenergie des ersten Energiebereichs und die zweite, untere Grenzenergie des zweiten Energiebereichs mittels einer anpassbaren Energieschwelle angepasst werden. Die jeweiligen Grenzenergien können jedoch nach der Anpassung auch unterschiedliche Energiewerte annehmen. Insbesondere kann die erste, oberer Grenzenergie des ersten Energiebereichs auf den gleichen oder einen niedrigeren Energiewert angepasst werden als die zweite, untere Grenzenergie des zweiten Energiebereichs.

Die Anpassung in Abhängigkeit des ersten und des zweiten Energiespektrums kann umfassen, dass die zumindest eine Grenzenergie in Hinblick auf eine optimierte Bildqualität, insbesondere in Hinblick auf ein Bildrauschen, ein Bildkontrast oder einem Auftreten von Artefakten im mittels des ersten und des zweiten Energiespektrums erzeugten CT-Bilddatensatz, angepasst wird.

Das Anpassen basiert dabei auf einem optimierten Energiewert für die jeweilige zumindest eine Grenzenergie. Der optimierten Energiewert für die jeweilige zumindest eine Grenzenergie kann demjenigen Energiewert entsprechen, welcher zu einer gewünschten bzw. zur Beantwortung einer klinischen Fragestellung notwendigen Bildqualität führt, wenn der Röntgenbilddatensatz mit darauf basierenden angepassten Energiebereichen erzeugt wird.

Der optimierte Energiewert kann anhand eines Kriteriums, d.h. anhand eines Optimierungskriteriums, optimiert sein, umfassend, unter anderem, beispielsweise ein Bildrauschwert des CT-Bilddatensatzes oder ein Bildkontrastwert des CT-Bilddatensatzes. Der optimierte Energiewert kann beispielsweise hinsichtlich eines Kontrast-zu-Rausch Verhältnisses oder eines Signal-zu-Rausch-Verhältnisses optimiert sein. Das heißt, durch Anwenden des optimierten Energiewerts kann beispielsweise ein vorteilhaft niedriges Rauschlevel im erzeugten CT-Bilddatensatzes ermöglicht werden. Der optimierte Energiewert kann auch hinsichtlich eines besonders vorteilhaften Materialkontrasts zwischen zwei Materialien optimiert sein. Das kann bedeuten, dass durch Anwenden des optimierten Energiewerts eine Unterscheidbarkeit oder Separierung zweier Materialien, beispielsweise Jod und Gewebe mit einem wasserähnlichen Absorptionsverhalten von Röntgenstrahlung, im erzeugten CT-Bilddatensatzes besonders möglich ist.

Das Optimierungskriterium kann auch ein Artefaktwert im CT-Bilddatensatzes sein, welcher mit dem Auftreten eines Artefakts verknüpft ist. Der optimierte Energiewert kann beispielsweise hinsichtlich der Vermeidung oder Reduzierung eines oder mehrerer Artefakte im Röntgenbilddatensatz optimiert sein. Das kann bedeuten, dass durch Anwenden des optimierten Energiewerts ein Artefakt vermindert im erzeugten Röntgenbilddatensatz auftritt.

Das Optimierungskriterium kann den spektralen Überlapp zwischen dem ersten Energiespektrum und dem zweiten Energiebereich und dem zweiten Energiespektrum und dem ersten Energiebereich umfassen. Insbesondere kann der optimierte Energiewert hinsichtlich eines minimalen spektralen Überlapp bei gleichzeitig möglichst guter Dosisausnutzung optimiert sein. Das bedeutet beispielsweise, dass die erste, obere Grenzenergie derart gesetzt wird, dass der erste Energiebereich möglichst wenig mit dem zweiten Energiespektrum überlappt, jedoch gleichzeitig möglichst wenig Photonen des ersten Energiespektrums nicht für die Erzeugung des CT-Bilddatensatzes genutzt werden. Äquivalentes gilt für den zweiten Energiebereich. Durch Minimierung des spektralen Überlapp der Energiebereiche und Energiespektren kann verbessert eine spektrale Separierung der auf den Messsignalen basierenden Datensätzen ermöglicht werden. Dadurch können besonders hochqualitative Röntgenbilddatensätze mittels einer spektralen Datenverarbeitungstechnik erzeugt werden können.

Ein bereits ermittelter, optimierte Energiewert kann für den Schritt des Anpassens beispielsweise bereits in Form einer Datenbank auf einem Datenspeicher vorliegen und für den Schritt des Anpassens mittels einer Schnittstelle durch die Anpassungseinheit abrufbar sein. Der optimierte Energiewert kann jedoch auch in zeitlicher Nähe zum Schritt des Anpassens ermittelt werden.

Der optimierte Energiewert kann beispielsweise mittels eines maschinellen Lernverfahrens oder auch mittels eines analytischen Verfahrens optimiert sein. Beispielsweise kann eine Ermittlung auf einer Vielzahl an gemessenen oder simulierten (Trainings-)Bilddatensätzen basieren, welche jeweils auf in unterschiedliche Energiebereiche aufgelöste Messignale basieren. Durch einen Vergleich der unterschiedlichen Bilddatensätze in Abhängigkeit der Energiebereiche bzw. daraus abgeleiteter Kriterien, wie etwa einem Bildrauschwert, einen Bildkontrastwert oder einem Artefaktwert, kann dann ein optimierter Energiewert für die jeweilige zumindest eine Grenzenergie ermittelt werden. Es ist jedoch auch denkbar, dass der optimierte Energiewert lediglich anhand des ersten und des zweiten Energiespektrums ermittelt wird. Die Anpassung in Abhängigkeit des ersten und des zweiten Energiespektrums kann dann umfassen, dass die zumindest eine Grenzenergie des ersten Energiebereichs, insbesondere die erste, obere Grenzenergie in Abhängigkeit der maximalen, emittierten Energie des ersten Energiespektrums und der minimalen, emittierten Energie des zweiten Energiespektrums angepasst wird. Ebenso ist denkbar, dass eine Anpassung in Abhängigkeit der mittleren Energie des ersten und/oder zweiten Energiespektrums, einem Integral über zumindest einen Teil des ersten und/oder zweiten Energiespektrums, einer stark ansteigenden oder stark abfallenden Flanke des ersten und/oder zweiten Energiespektrums angepasst wird. Dies kann ebenso für die zumindest eine Grenzenergie des zweiten Energiebereichs, insbesondere die zweite, niederenergetischere Grenzenergie, gelten.

Beispielsweise ist dabei eine Ausführungsvariante denkbar, dass durch einen Anwender eine Information über das erste und das zweite Energiespektrum mittels einer Eingabeeinheit eingegeben werden kann, worauf basierend der optimierte Energiewert abgerufen oder ermittelt wird. Die Information kann beispielsweise die Röhrenspannung einer verwendeten Röntgenröhre oder einen verwendeten Vorfilter umfassen. Ebenso ist denkbar, dass eine bestimmte medizinische Untersuchungsart mit einem ersten und einem zweiten Energiespektrum verknüpft ist und basierend auf einer Eingabe einer medizinischen Untersuchungsart der optimierte Energiewert abgerufen oder ermittelt wird. Auch ist denkbar, dass direkt aus der emittierten ersten und zweiten Röntgenstrahlung Information über das erste und das zweite Energiespektrum abgleitet werden kann, worauf basierend der optimierte Energiewert abgerufen oder ermittelt werden kann.

Das Anpassen der Energiebereiche kann vollautomatisch mittels der Anpassungseinheit durchgeführt werden, sobald beispielsweise Informationen über die Untersuchungsart oder die Energiespektren vorliegen. Das Anpassen kann eine Bestätigung durch einen Anwender umfassen, wobei ein Anpassungsvorschlag, beispielsweise vorgeschlagene Energiebereiche bzw. Grenzenergien, erst nach der Bestätigung umgesetzt wird. Beispielsweise wird nach einer Vorgabe des erste und des zweiten Energiespektrums die jeweilige zumindest eine Grenzenergie voll- oder zumindest teil-automatisch mittels der Anpassungseinheit angepasst.

Im erfindungsgemäßen Schritt des Emittierens wird Röntgenstrahlung aufweisend das erste Energiespektrum und aufweisend das zweite Energiespektrum mittels der Röntgenquelleneinheit emittiert. Das erste und das zweite Energiespektrum kann zeitgleich oder zeitlich versetzt mittels einer oder mehr Röntgenquellen, beispielsweise Röntgenröhren, emittiert werden. Eine Röntgenquelle ist jeweils ausgebildet die Röntgenstrahlung in Richtung eines ihr zugeordneten Röntgendetektors zu emittieren.

Im Schritt des Detektierens wird die emittierte Röntgenstrahlung aufweisend das erste Energiespektrum und aufweisend das zweite Energiespektrum mittels der Detektionseinheit, insbesondere nach Durchgang durch ein Untersuchungsobjekt detektiert, wobei zumindest ein erstes Messsignal in Abhängigkeit des ersten Energiebereichs und des ersten Energiespektrums und zumindest ein zweites Messsignal in Abhängigkeit des zweiten Energiebereichs und des zweiten Energiespektrums generiert wird. Das erste Messsignal und das zweite Messignal kann von demselben photonenzählenden Röntgendetektor generiert werden. Im Falle von zwei photonenzählenden Röntgendetektoren kann auch das erste Messignal von dem ersten der zwei photonenzählenden Röntgendetektoren und das zweite Messsignal von dem zweiten der zwei photonenzählenden Röntgendetektoren generiert werden. Neben dem ersten und dem zweiten Messsignal können darüber hinaus auch weitere Messsignale generiert werden.

Der Schritt des Emittierens und des Detektierens findet im Wesentlichen zeitgleich während einer Rotationsbewegung der Detektionseinheit und der Röntgenquelleneinheit um das Untersuchungsobjekt statt. Derart können jeweils erste und zweite Messsignale aus unterschiedlichen Winkelsektoren aufgenommen werden, worauf basierend der spektrale CT-Bilddatensatz erzeugt werden kann.

Durch die Bereitstellung des ersten Messsignal in Abhängigkeit des ersten Energiespektrums und durch Bereitstellung des zweiten Messsignals in Abhängigkeit des zweiten, vom ersten verschiedenen Energiespektrums wird die Anwendung einer spektralen Bildverarbeitungstechnik für die Erzeugung des Röntgenbilddatensatzes verbessert ermöglicht. Im erfindungsgemäßen Schritt des Erzeugens wird der spektrale CT-Bilddatensatz entsprechend basierend zumindest auf dem generierten ersten und dem generierten zweiten Messsignal mit Hilfe einer spektralen Bildverarbeitungstechnik von einer Bildverarbeitungseinheit erzeugt. Insbesondere kann das Erzeugen auf einer Vielzahl an ersten Messignalen und einer Vielzahl an zweiten Messsignalen basieren, welche jeweils aus unterschiedlichen Winkelsektoren aufgenommen wurden. Auf dem Gebiet der CT-Bildverarbeitung sind dem Fachmann dabei eine Vielzahl an spektraler Bildverarbeitungstechniken bekannt, beispielsweise zur Berechnung von Basismaterialbildern, zur Berechnung von pseudo-monoenergetischen Bildern, oder zur Klassifizierung von Materialien im CT-Bilddatensatz, so dass diese hier nicht weiter ausgeführt werden sollen. Anschließend wird der erzeugte spektralen CT-Bilddatensatz erfindungsgemäß mittels einer Schnittstelle ausgegeben. Der Röntgenbilddatensatz kann an eine Darstellungseinheit zur Darstellung des Röntgenbilddatensatzes und beispielsweise einer darauf basierenden Befundung für einen Anwender ausgegeben werden. Der Röntgenbilddatensatz kann auch an eine Weiterverarbeitungseinheit ausgegeben werden, welche den Datensatz weiterverarbeitet. Beispielsweise können darauf basierend Strukturen segmentiert oder vermessen werden.

Der jeweilige Einsatz der oben genannten Verfahren (Dual Source, kV-Switching, Split-Filter, energieauflösender Detektor) zu Aufnahme spektraler CT-Messdaten allein lässt sich eine energetische Separierung der aufgenommenen Messdatensätze nur ungenügend erreichen. Bei bekannten Verfahren, die auf der Aufnahme von zwei CT-Datensätzen mit unterschiedlicher Röhrenspannung basieren wie etwa "Dual Source" Systeme oder "kV-Switching" Systeme in Kombination mit einem integrierenden Detektor, ergibt sich zwar eine unterschiedliche mittlere Energie der beiden Eingangsdatensätze, jedoch ein erheblicher Überlapp der beiden Datensätze im Energiebereich bis zur Maximalenergie des mit der niedrigeren Röhrenspannung aufgenommenen Datensatzes. Bei Verfahren mit "Dual Layer" Detektoren ergibt sich ein spektraler Überlapp der beiden Datensätze im gesamten Energiebereich. Auch der konventionelle Einsatz eines photonenzählenden Detektors mit zwei oder mehr Energietöpfen liefert keine energetisch scharf in diese Energiebereiche getrennte Daten. Die in den einzelnen Energietöpfen aufgenommenen CT-Messdaten zeigen aufgrund physikalischer Effekte wie charge sharing ("Ladungsteilung" eines Events) zwischen benachbarten Detektorelementen oder Fluoreszenz im Detektormaterial einen spektralen Überlapp, dadurch gekennzeichnet, dass Röntgenquanten mit höherer Energie fälschlicherweise mit zu niedriger Energie, also in niederenergetischen Energietöpfen registriert werden. Der spektrale Überlapp kann die Qualität spektraler Algorithmen verschlechtern, das Bildrauschen vergrößern und Artefakte der mit diesen Algorithmen erzeugten Ergebnisbilder verstärkt auftreten lassen. Spektraler Überlapp führt letztlich zu deutlichen Beschränkungen und höheren Strahlendosisanforderungen für spektrale CT-Untersuchungen.

Die Erfinder haben erkannt, dass durch die Kombination einer Detektionseinheit aufweisend zumindest einen photonenzählenden Röntgendetektor und ausgebildet detektierte Röntgenstrahlung in Messsignale umzuwandeln, welche zumindest in einen ersten, anpassbaren Energiebereich und einen zweiten, anpassbaren Energiebereich aufgelöst sind, mit einer Röntgenquelleneinheit, welche ausgebildet Röntgenstrahlung aufweisend ein erstes Energiespektrum und aufweisend ein zweites, vom ersten verschiedenes Energiespektrum zu emittieren, eine verbesserte spektrale Trennung ermöglicht werden kann, indem in vorteilhafter Weise ein erstes Messsignal in Abhängigkeit des ersten Energiebereichs und des ersten Energiespektrums und ein zweites Messignal in Abhängigkeit des zweiten Energiespektrums und des zweiten Messsignals für die Erzeugung bereitgestellt werden können. Dabei ist vorteilhaft der erste und der zweite Energiebereich an das erste und das zweite Energiespektrum angepasst. Das erfindungsgemäße Verfahren ermöglicht dadurch vorteilhaft die Erzeugung von qualitativ hochwertigeren, spektralen CT-Bilddatensätzen, indem optimierte Messsignale, d.h. für die spektrale Datenverarbeitungstechnik optimierte Datensätze, bereitgestellt werden können.

In einer vorteilhaften Ausgestaltung der Erfindung kann vorgesehen sein, dass im Schritt des Anpassens die jeweilige zumindest eine Grenzenergie des ersten Energiebereichs und des zweiten Energiebereichs in Abhängigkeit der spektralen Bildverarbeitungstechnik, einer medizinischen Untersuchungsart und/oder einer patientenspezifischen Information automatisch mittels der Anpassungseinheit angepasst wird.

Dieser Aspekt der Erfindung beruht auf der Überlegung, dass die Bildqualität des Bilddatensatz dadurch optimiert werden kann, das unterschiedliche Bildverarbeitungstechnik gegebenenfalls unterschiedliche Anforderungen an die bereitgestellten Messignale stellen können. Eine Anpassung der Energiebereiche bzw. der jeweiligen zumindest einen Grenzenergie des ersten und des zweiten Energiebereichs in Abhängigkeit der spektralen Bildverarbeitungstechnik kann dann vorteilhaft zu einer verbesserten Bildqualität führen.

Ebenso haben die Erfinder erkannt, dass die Bildqualität des erzeugten Bilddatensatz dadurch optimiert werden kann, dass Energiebereiche unter Berücksichtigung der Art der Untersuchung angepasst werden, da unterschiedliche Untersuchungsarten gegebenenfalls unterschiedliche Anforderungen an die Röntgenbildaufnahme bzw. an die bereitgestellten Messignale stellen können. Eine Untersuchungsart kann außerdem mit verwendeten Energiespektren oder mit einer verwendeten spektrale Datenverarbeitungstechnik verknüpft sein, worauf basierend vorteilhart eine optimierte Anpassung mittels der Anpassungseinheit erfolgen kann.

Unter Untersuchungsart ist dabei jede beliebige medizinische oder klinische Fragestellung zu verstehen, die anhand von Röntgenbildaufnahmen beantwortet werden kann. Beispielsweise kann eine Untersuchung von Blutgefäßen mittels einer Angiographie-Aufnahme oder eine Untersuchung des Leberparenchyms, jeweils unter Kontrastmittelgabe, oder einer Untersuchung von Knochengewebe ohne Kontrastmittel erfolgen. Die Anpassung kann dann in Abhängigkeit der Untersuchungsart, insbesondere der geplanten Untersuchungsart erfolgen, die anhand des erzeugten CT-Bilddatensatz für den Patienten erfolgen soll.

Ebenso haben die Erfinder erkannt, dass die Bildqualität des erzeugten CT-Bilddatensatz auch dadurch optimiert werden kann, dass Energiebereiche unter Berücksichtigung einer patientenspezifischen Information angepasst werden. Dieser Aspekt der Erfindung basiert auf der Überlegung, dass der Patient, d.h. das Untersuchungsobjekt, durch die Abschwächung der Röntgenstrahlung im Patienten, Einfluss auf das Energiespektrum hinter dem Patienten haben kann und damit für unterschiedliche Patienten oder unterschiedliche Bereiche des Patienten gegebenenfalls unterschiedlich angepasste Energiebereiche vorteilhaft für die Erzeugung sind. Darüber hinaus erfolgt häufig eine Auswahl der emittierten Energiespektren basierend auf patientenspezifischen Informationen, so dass hierüber Informationen über die eingesetzten Energiespektren abgeleitet werden können, welche für die automatische Anpassung der Energiebereiche vorteilhaft genutzt werden können.

Eine patientenspezifische Information kann eine patientenspezifische, körperbezogene Information über den Patienten sein, von dem ein Computertomographie-Bilddatensatz erzeugt werden soll. Die patientenspezifische Information kann beispielsweise eine der folgenden Informationen umfasst: Patientengröße, Patientenbreite, Patientenform, Patientengewicht und/oder Röntgenschwächungsverhalten des Patienten. Wenigstens eine oder mehrere patientenspezifische Informationen können sich beispielsweise durch die Auswertung eines Patienten-Topograms, einer photographischen Abbildung des Patienten oder einer oder mehreren Röntgenbildaufnahmen des Patienten, die beispielsweise von in der Vergangenheit liegenden Röntgenuntersuchungen stammen. Alternativ kann beispielsweise eine patientenspezifische Information über den Patienten ermittelt werden, indem eine Waage in die Patientenliege der Röntgenbildaufnahmevorrichtung zur Bestimmung des Patientengewichtes integriert ist. In einer weiteren Ausführung kann eine Eingabe von patientenspezifischen, körperbezogenen Informationen über eine Eingabeeinheit durch einen Benutzer erfolgen.

Beispielsweise können konkrete Energiewerte für die jeweilige zumindest eine Grenzenergie oder ein erstes und zweites Energiespektrum, oder abrufbare Funktionen in Form von arithmetischen Funktionen oder in Form von maschinellen Lernsystemen zur Ermittlung von Energiewerten verknüpft mit einer Auswahl an spektralen Bildverarbeitungstechniken, medizinischen Untersuchungsarten und/oder patientenspezifischen Informationen oder darauf abgeleiteter Informationen vorliegen. Im Rahmen einer spektralen CT-Untersuchung kann dann eine konkrete spektrale Bildverarbeitungstechnik, eine medizinische Untersuchungsart und/oder eine patientenspezifische Information vorgegeben oder ermittelt werden. Darauf basierend kann jeweils ein Energiewert für die jeweilige zumindest eine Grenzenergie abgerufen oder mittels einer abrufbaren Funktion ermittelt werden und die Energiebereiche automatisch mittels der Anpassungseinheit angepasst werden.

Eine Ausgestaltung im Rahmen der Erfindung kann entsprechend vorsehen, dass ein Anwender, d.h. Benutzer, vor der Erzeugung des CT-Bilddatensatzes ein erstes oder zweites Energiespektrum, eine spektrale Datenverarbeitungstechnik, eine Untersuchungsart oder patientenspezifische Information vorgibt bzw. mittels einer dazu ausgebildeten Eingabeeinheit eingibt.

Erfindungsgemäß umfasst das Verfahren außerdem den Schritt des Ermittelns eines optimierten Energiewerts für die jeweilige zumindest eine Grenzenergie des ersten Energiebereichs und des zweiten Energiebereichs zumindest basierend auf dem ersten und dem zweiten Energiespektrum mittels einer Optimierungseinheit, wobei der optimierte Energiewert anhand eines Kriteriums der folgenden Liste optimiert wird
- Ein Bildrauschwert des CT-Bilddatensatzes
- Einen Bildkontrastwert des CT-Bilddatensatzes
- Ein Artefaktwert des CT-Bilddatensatzes
- Ein Wert des spektralen Überlapp.

Dabei kann auch die spektralen Bildverarbeitungstechnik, einer medizinischen Untersuchungsart und/oder patientenspezifische Informationen in das Ermitteln miteingehen.

Der Schritt des Ermittelns kann dabei das Anwenden eines maschinellen Lernverfahrens umfassen. Der Schritt des Ermittelns kann auch auf einem arithmetischen Verfahren beruhen. Das Ermitteln kann mittels eines künstlichen Intelligenzsystems, d.h. durch ein Verfahren des maschinellen Lernens, realisiert sein. Unter einem künstlichen Intelligenzsystem kann man ein System für die künstliche Generierung von Wissen aus Erfahrung bezeichnen. Ein künstliches System lernt aus Beispielen in einer Trainingsphase und kann nach Beendigung der Trainingsphase verallgemeinern. Die Verwendung eines solchen Systems kann ein Erkennen von Mustern und Gesetzmäßigkeiten in den Trainingsdaten umfassen. Das künstliche Intelligenzsystem kann ein künstliches neuronales Netz, insbesondere ein faltendes neuronales Netz oder auch auf einem anderen Verfahren des maschinellen Lernens basieren. Insbesondere können mittels eines künstlichen Intelligenzsystems nach der Trainingsphase eine optimierte Grenzenergie besonders verlässlich und zeiteffizient automatisiert identifiziert werden.

Beispielsweise kann eine Ermittlung auf einer Vielzahl an gemessenen oder simulierten (Trainings-)Bilddatensätzen basieren, welche jeweils auf in unterschiedliche Energiebereiche aufgelöste Messignale basieren. Durch einen Vergleich der unterschiedlichen Bilddatensätze bzw. daraus abgeleiteter Kriterien, wie etwa einem Bildrauschwert, einen Bildkontrastwert oder einem Artefaktwert, kann dann ein optimierter Energiewert für die jeweilige zumindest eine Grenzenergie ermittelt werden.

Vorteilhaft kann ein optimierter Energiewert für das Anpassen bereitgestellt werden.

Dabei kann das Ermitteln auch zeitlich unabhängig und für eine Vielzahl von ersten und zweiten Energiespektren bereits im Vorfeld durchgeführt werden. Als Ergebnis kann dann beispielsweise eine Datenbank bereitgestellt werden, welche in Abhängigkeit des ersten und zweiten Energiespektrum, der spektralen Bildverarbeitungstechnik, der medizinischen Untersuchungsart und/oder patientenspezifischer Informationen jeweilige optimierte Energiewerte in Form einer Datenbank, beispielsweise einer Tabelle oder ähnlichem, für die Anpassungseinheit abrufbar bereitstellt. Darüber hinaus sind auch weitere Parameter denkbar.

In einer Ausführungsvariante können für den Schritt des Ermittelns trainierte bzw. angepasste Funktionen abrufbar bereitgestellt sein, welche in Abhängigkeit des ersten und zweiten Energiespektrum, der spektralen Bildverarbeitungstechnik, einer medizinischen Untersuchungsart und/oder einer patientenspezifische Information oder auch andere Parameter als Eingangsparameter den optimierten Energiewert ermitteln und an die Anpassungseinheit ausgeben.

Eine vorteilhafte Weiterbildung der Erfindung sieht dabei vor, dass der erste Energiebereich an den zweiten Energiebereich angrenzt.

In dieser Konfiguration nimmt die jeweils zumindest eine Grenzenergie des ersten und des zweiten Energiebereichs einen gemeinsamen Energiewert an. Dies stellt eine besonders einfache Konfiguration hinsichtlich des ersten und zweiten Energiebereichs dar. Vorteilhaft ist eine solche Konfiguration bereits mit einem einzelnen photonenzählenden Röntgendetektor bereitstellbar, welcher lediglich zwei Energieschwellen für die Anpassung der Energiebereiche bereitstellt. Ebenso kann dadurch vorteilhaft eine Vereinfachung einer Ermittlung eines optimierten Energiewerts durch Reduzierung der Parameter erreicht werden.

In einer anderen Ausführung der Erfindung kann der erste Energiebereich und der zweite Energiebereich voneinander beabstandet sind.

Beabstandet bedeutet, dass die erste, obere Grenzenergie des ersten Energiebereichs einen anderen, insbesondere geringeren Energiewert annimmt als die zweite, unterer Grenzenergie des zweiten Energiebereichs, so dass zwischen dem ersten Energiebereich und dem zweiten Energiebereich ein weiterer Energiebereich liegt, welcher den ersten und den zweiten Energiebereich voneinander separiert.

Vorteilhaft kann dadurch eine verbesserte spektrale Separierung der generierten Messsignale ermöglicht werden, indem beispielsweise zumindest zum Teil ein Energiebereich, in welchem das erste Energiespektrum gegebenenfalls mit dem zweiten Energiespektrum überlappt für die Bilderzeugung ausgespart wird oder zumindest weniger stark gewichtet in die Erzeugung miteingeht, wohingegen Energiebereiche mit wenig oder keinem spektralen Überlapp den wesentlichen Beitrag zur Erzeugung des spektralen CT-Röntgenbilddatensatzes liefern.

In einer einfachen Variante des erfindungsgemäßen Verfahrens geht lediglich erste Messignale in Abhängigkeit des ersten Energiespektrums und des ersten Energiebereichs und zweite Messsignale in Abhängigkeit des zweiten Energiespektrums und des zweiten Energiebereichs in die Erzeugung des spektralen Röntgenbilddatensatz ein um eine verbesserte spektrale Separierung des Datensatzes basierend auf dem ersten Energiespektrum und des Datensatzes basierend auf dem zweiten Energiespektrum zu erreichen.

Weiterhin ist jedoch in einer weiteren vorteilhaften Weiterbildung vorgesehen, insbesondere in dem Fall, dass der erste Energiebereich an den zweiten Energiebereich angrenzt, außerdem ein drittes Messsignal in Abhängigkeit des ersten Energiespektrums und des zweiten Energiebereichs und ein viertes Messsignal in Abhängigkeit des zweiten Energiespektrums und des ersten Energiebereichs generiert werden und wobei im Schritt des Erzeugens außerdem auch das dritte und vierte Messsignal eingehen.

Ebenso kann, insbesondere in dem Fall, dass der erste Energiebereich und der zweite Energiebereich voneinander beabstandet ist, außerdem ein drittes Messsignal in Abhängigkeit des ersten Energiespektrums und eines dritten Energiebereichs und ein viertes Messsignal in Abhängigkeit des zweiten Energiespektrums und eines vierten Energiebereichs generiert werden und wobei im Schritt des Erzeugens außerdem auch das dritte und vierte Messsignal eingehen. Der dritte Energiebereich kann beispielsweise einen an den ersten Energiebereich angrenzenden, höherenergetischen Energiebereich umfassen, welcher sich vom zweiten Energiebereich unterscheidet. Der vierte Energiebereich kann beispielsweise einen an den zweiten Energiebereich angrenzenden, niederenergetischeren Energiebereich umfassen, welcher sich jedoch von dem ersten Energiebereich unterscheidet. Es sind darüber hinaus auch weitere Konfigurationen an Energiebereichen möglich.

Vorteilhaft können durch Eingang der dritten und vierten Messsignale möglichst die gesamte zur Verfügung stehende Dosisinformationen für die Erzeugung des CT-Röntgenbilddatensatzes genutzt werden und damit gegebenenfalls eine unnötige Strahlenbelastung eines Patienten vermieden werden.

Bevorzugter Weise können die generierten Messsignale dabei mittels Wichtungsfaktoren gewichtet in die Erzeugung des CT-Bilddatensatzes eingehen, wobei das erste Messsignal insbesondere höher gewichtet wird als das dritte Messsignal und das zweite Messsignal insbesondere höher gewichtet wird als das vierte Messsignal.

Vorteilhaft kann eine verbesserte Quantenausnutzung bei gleichzeitig möglichst guter spektraler Trennung der Messdatensätze erreicht werden.

Dabei können insbesondere optimierte Gewichtungsfaktoren zum Einsatz kommen, die basierend einem Bildrauschwert des Röntgenbilddatensatzes, einem Bildkontrastwert des Röntgenbilddatensatzes oder einem Artefaktwert des Röntgenbilddatensatzes optimiert sind. Die optimierten Wichtungsfaktoren für die jeweiligen Messsignale können denjenigen Wichtungsfaktoren entsprechen, welcher zu einer gewünschten bzw. zur Beantwortung einer klinischen Fragestellung notwendigen Bildqualität führen, wenn der Röntgenbilddatensatz darauf basieren erzeugt wird. Die optimierten Gewichtungsfaktoren können beispielsweise mittels eines Verfahrens des maschinellen Lernens oder auch mittels eines anderen Verfahrens optimiert sein.

In weiteren vorteilhaften Ausbildungen können darüber hinaus auch weitere Energiebereiche vorgesehen sein und dementsprechend weitere Messsignale generiert werden. Es sind unterschiedlichste Konfigurationen mit einer Auflösung in zwei, drei, vier, fünf oder mehr Energiebereiche denkbar, deren Messsignale vorzugsweise mittels Wichtungsfaktoren gewichtet in die Erzeugung miteingehen können. Eine Auflösung in mehr als zwei Energiebereiche kann eine feinere Abstimmung der Energiebereiche auf das erste und das zweite Energiespektrum und eine feinere Abstimmung der Gewichte der Messsignale im Schritt des Erzeugens ermöglichen. Dadurch kann die spektrale Separation bei gleichzeitig bestmöglicher Quantennutzung der applizierten Röntgenstrahlen gegebenenfalls verbessert werden.

In einer weiteren vorteilhaften Ausgestaltungsvariante des erfindungsgemäßen Verfahrens wird im Schritt des Emittierens selektiv abwechselnd entweder das erste Energiespektrum oder das zweite Energiespektrum emittiert.

Vorteilhaft kann das Verfahren mittels lediglich eines Röntgendetektors und einer Röntgenquelle realisiert werden. Diese Variante des erfindungsgemäßen Verfahrens kann insbesondere dadurch realisiert werden, indem die Röntgenquelleneinheit eine Röntgenquelle vorsieht, welche für eine Methode des sogenannten kV Switchings ausgebildet ist. Nachteil einer solchen Konfiguration ist die relativ kostenintensive Bereitstellung einer dafür ausgebildeten Röntgenquelle.

Weiterhin kann in einer vorteilhaften Variante vorgesehen sein, dass die Detektionseinheit einen photonenzählenden Röntgendetektor mit einer Mehrzahl an Detektionselementen aufweist, und wobei eine erste Teilzahl der Mehrzahl an Detektionselementen mittels der Röntgenquelleneinheit mit dem ersten Energiespektrum belichtet wird und eine zweite Teilzahl der Mehrzahl mit dem zweiten Energiespektrum belichtet wird und wobei mittels der ersten Teilzahl der Mehrzahl an Detektionselementen das erste Messignal und mittels der zweiten Teilzahl der Mehrzahl an Detektionselementen das zweite Messignal generiert wird.

Vorteilhaft kann das Verfahren mittels lediglich eines Röntgendetektors und einer Röntgenquelle realisiert werden. Insbesondere kann diese Variante des erfindungsgemäßen Verfahrens mit einer Röntgenquelle aufweisend einen Split-Filter realisiert werden. Dies kann einer kostengünstigen Variante der Röntgenquelle entsprechen. Nachteil ist jedoch die geringere Einstellbarkeit der emittierten Energiespektren und der resultierende großer Überlapp der Energiespektren bis zur maximal emittierten Energie. Jedoch kann auch hier mittels des Verfahrens eine verbesserte Bildqualität durch die Bereitstellung des ersten und des zweiten Messsignals und einer Anpassung der Energiebereiche in Abhängigkeit des ersten Energiespektrums und des zweiten Energiespektrums vorteilhaft erreicht werden.

Daneben kann in einer besonders vorteilhaften Ausgestaltung des erfindungsgemäßen Verfahrens die Detektionseinheit einen ersten photonenzählenden Röntgendetektor und einen winkelversetzt dazu angeordneten, zweiten photonenzählenden Röntgendetektor aufweisen, und die Röntgenquelleneinheit jeweils in Gegenüberstellung dazu eine erste Röntgenquelle, welche das erste Energiespektrum emittiert, und eine zweite Röntgenquelle, welche das zweite Energiespektrum emittiert, aufweisen, wobei mittels des ersten Röntgendetektors das erste Messsignal und mittels des zweite Röntgendetektors das zweite Messignal generiert wird.

Vorteilhaft sind eine hohe Flexibilität und eine gute spektrale Trennung der generierten Messdaten erreichbar. Darüber hinaus kann durch Bereitstellung von zwei photonenzählenden Röntgendetektoren auch bei gleicher Anzahl an Energieschwellen eine hohe Variabilität und Anpassbarkeit der Energiebereiche ermöglicht werden, da die Röntgendetektoren und deren Energiebereiche jeweils gesondert voneinander angepasst werden können.

Die Erfindung betrifft außerdem eine Vorrichtung zur Erzeugung eines spektralen CT-Bilddatensatzes gemäss Anspruch 12.

Die Vorrichtung umfasst eine Optimierungseinheit (25), ausgebildet zum Ermitteln (S0) eines optimierten Energiewerts für die jeweilige zumindest eine Grenzenergie (GE1, GE2) des ersten Energiebereichs (EB1) und des zweiten Energiebereichs (EB2) zumindest basierend auf dem ersten Energiespektrum (9) und dem zweiten Energiespektrum (10) mittels einer Optimierungseinheit (25), wobei außerdem zumindest ein Kriterium der folgenden Liste optimiert wird
- Ein Bildrauschwert des Computertomographie-Bilddatensatzes,
- Ein Bildkontrastwert des Computertomographie-Bilddatensatzes,
- Ein Materialkontrastwert des Computertomographie-Bilddatensatzes,
- Ein Artefaktwert des Computertomographie-Bilddatensatzes,Ein Wert des spektralen Überlapp zwischen dem ersten Energiespektrum (9) und dem zweiten Energiebereich (EB2) und/oder dem zweiten Energiespektrum (10) und dem ersten Energiebereich (EB1).

Die erfindungsgemäße Vorrichtung zur Erzeugung eines spektralen Röntgenbilddatensatzes kann insbesondere dazu ausgebildet sein die zuvor beschriebenen erfindungsgemäße Verfahren und ihre Aspekte auszuführen. Die Vorrichtung zur Erzeugung eines spektralen Röntgenbilddatensatzes kann dazu ausgebildet sein die Verfahren und ihre Aspekte auszuführen, indem die Röntgenquelleneinheit, die Detektionseinheit, die Anpassungseinheit, die Bildverarbeitungseinheit und die Schnittstelle ausgebildet sind, die entsprechenden Verfahrensschritte auszuführen.

Die Schnittstelle kann insbesondere ausgebildet sein den spektralen CT-Bilddatensatz an eine Ausgabeeinheit, beispielsweise ein Display zur Darstellung des Bilddatensatzes, auszugeben. Ebenso kann die Schnittstelle ausgebildet sein den spektralen CT-Bilddatensatz an eine Weiterverarbeitungseinheit auszugeben.

Insbesondere kann die Vorrichtung außerdem auch eine Eingabeeinheit für eine Eingabe eines Benutzers umfassen, beispielsweise um Informationen über den ersten Energiebereich und den zweiten Energiebereich, der spektralen Bildverarbeitungstechnik, einer medizinischen Röntgenanwendung und/oder eines Untersuchungsobjekts einzugeben.

In einer vorteilhaften Ausgestaltung der erfindungsgemäßen Vorrichtung umfasst die Röntgenquelleneinheit eine Röntgenquelle mit einem Split-Filter, welche ausgebildet ist, gleichzeitig sowohl das erste Energiespektrum als auch das zweite Energiespektrum zu emittieren.

Alternativ kann die Röntgenquelleneinheit eine Röntgenquelle aufweisen, welche ausgebildet ist, selektiv abwechselnd zwischen dem ersten Energiespektrum und dem zweiten Energiespektrum umzuschalten.

Alternativ kann auch vorgesehen sein, dass die Detektionseinheit einen ersten photonenzählenden Röntgendetektor und einen winkelversetzt dazu angeordneten, zweiten photonenzählenden Röntgendetektor aufweist, und die Röntgenquelleneinheit jeweils in Gegenüberstellung dazu eine erste Röntgenquelle aufweist, ausgebildet das erste Energiespektrum zu emittieren, und eine zweite Röntgenquelle aufweist, ausgebildet das zweite Energiespektrum zu emittiert.

Im Folgenden wird die Erfindung anhand von beispielhaften Ausführungsformen unter Hinweis auf die beigefügten Figuren erläutert. Die Darstellung in den Figuren ist schematisch, stark vereinfacht und nicht zwingend maßstabsgetreu. Es zeigen:
Fig. 1 einen schematischen Ablauf eines Verfahrens zur Erzeugung eines spektralen Computertomographie-Bilddatensatzes,
Fig. 2 eine Darstellung eines beispielhaften ersten und zweiten Energiespektrums mit einer beispielhaften Ausführung eines ersten und zweiten Energiebereichs,

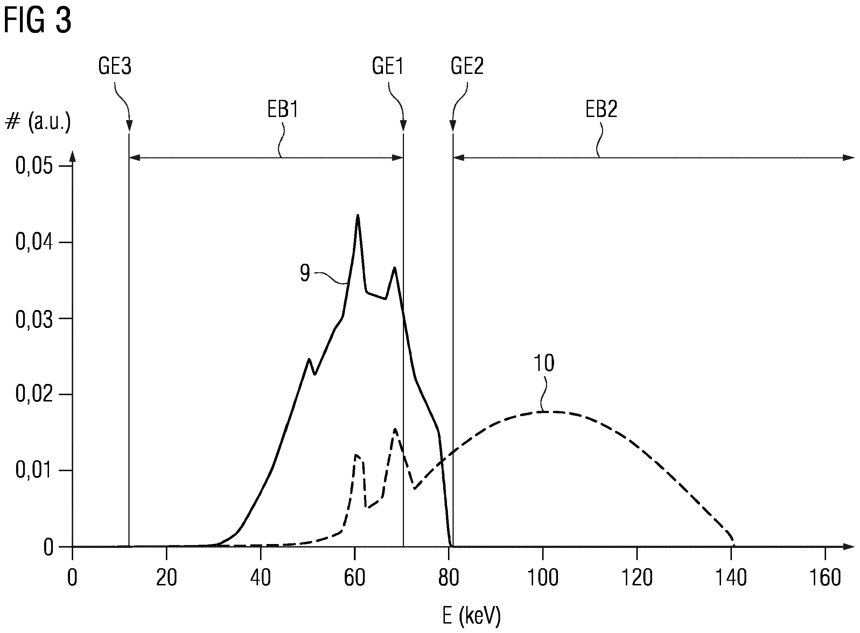

Fig. 3 eine Darstellung des beispielhaften ersten und zweiten Energiespektrums aus Fig. 2 mit einer weiteren beispielhaften Ausführung des ersten und zweiten Energiebereichs,

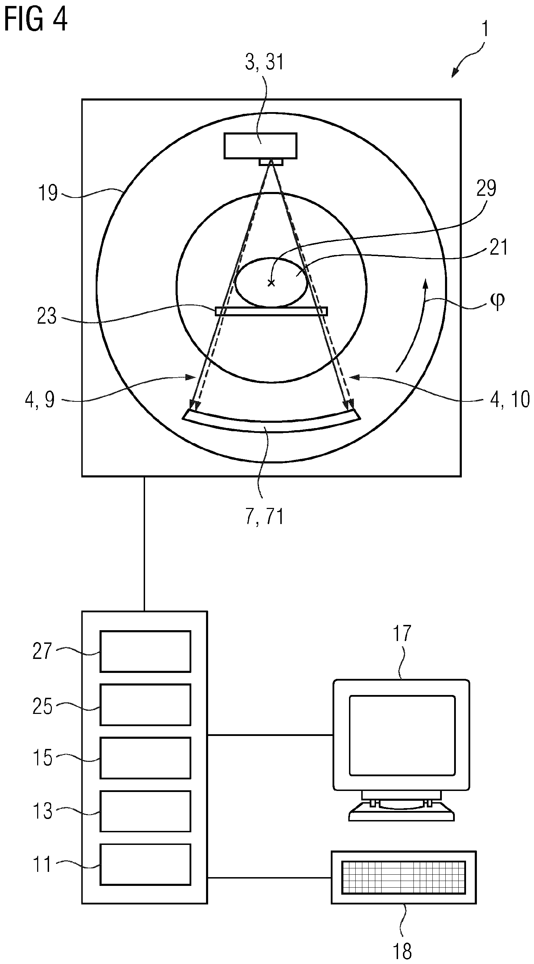

Fig. 4 eine schematische Darstellung einer beispielhaften Ausführungsform einer Vorrichtung zur Erzeugung eines spektralen Computertomographie-Bilddatensatzes,
Fig. 5 eine schematische Darstellung einer weiteren beispielhaften Ausführungsform einer Vorrichtung zur Erzeugung eines spektralen Computertomographie-Bilddatensatzes,
Fig. 6 eine schematische Darstellung einer Röntgenquelleneinheit und einer Detektionseinheit für den Einsatz in einer weiteren beispielhaften Ausführungsform der erfindungsgemäßen Vorrichtung zur Erzeugung eines spektralen Computertomographie-Bilddatensatzes.

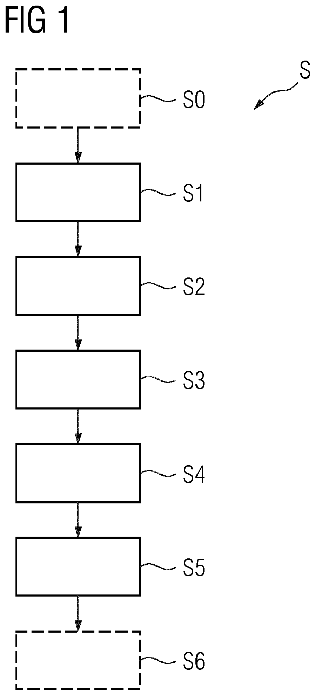

Fig. 1 zeigt einen schematischen Ablauf eines erfindungsgemäßen Verfahrens zur Erzeugung eines spektralen CT-Röntgenbilddatensatzes mittels einer Detektionseinheit 7, aufweisend zumindest einen photonenzählenden Röntgendetektor 71,72,73,74 und ausgebildet detektierte Röntgenstrahlung 4 in Messsignale umzuwandeln, welche zumindest in einen ersten, anpassbaren Energiebereich EB1 und einen zweiten, anpassbaren Energiebereich EB2 aufgelöst sind, und mittels einer Röntgenquelleneinheit 3, ausgebildet Röntgenstrahlung 4 aufweisend ein erstes Energiespektrum 9 und aufweisend ein zweites, vom ersten verschiedenes Energiespektrum 10 zu emittieren.

Das Verfahren weist den Schritt des Anpassens S1 auf. Dabei wird der erste Energiebereich EB1 und der zweite Energiebereich EB2 in Abhängigkeit des ersten Energiespektrums 9 und des zweiten Energiespektrums 10 mittels einer Anpassungseinheit 11 angepasst, wobei jeweils zumindest eine Grenzenergie GE1, GE2 eines jeweiligen Energiebereichs EB1, EB2 angepasst wird. Die jeweilige zumindest eine Grenzenergie GE1, GE2 eines jeweiligen Energiebereichs EB1, EB2 wird erfindungsgemäß dabei anhand eines optimierten Energiewerts angepasst. Insbesondere wird der optimierte Energiewert der jeweiligen zumindest eine Grenzenergie GE1, GE2 eines jeweiligen Energiebereichs EB1, EB2 in einem Schritt des Ermittelns S0 mittels einer Optimierungseinheit 25 ermittelt.

Das Ermitteln S0 des optimierten Energiewerts für die jeweilige zumindest eine Grenzenergie GE1, GE2 basiert zumindest auf dem ersten und dem zweiten Energiespektrum. Beim Schritt des Ermittelns S0 wird der optimierte Energiewert für die jeweilige zumindest eine Grenzenergie GE1, GE2 außerdem zumindest basierend auf einem Kriterium der folgenden Liste optimiert:
- Ein Bildrauschwert des CT-Bilddatensatzes
- Einen Bildkontrastwert des CT-Bilddatensatzes
- Ein Materialkontrastwert des CT-Bilddatensatzes
- Ein Artefaktwert des CT-Bilddatensatzes
- Ein Wert des spektralen Überlapp zwischen dem ersten Energiespektrum und dem zweiten Energiebereich und/oder dem zweiten Energiespektrum und dem ersten Energiebereich.

Der Schritt des Ermittelns S0 kann insbesondere auf einem Anwenden eines maschinellen Lernverfahrens, beispielsweise mittels eines trainierten neuronalen Netzes, basieren. Er kann aber auch anderweitig umgesetzt sein.

Im Schritt des Anpassens S1 kann außerdem vorgesehen sein, dass die jeweilige zumindest eine Grenzenergie GE1, GE2 des ersten Energiebereichs EB1 und des zweiten Energiebereichs EB2 außerdem in Abhängigkeit der spektralen Bildverarbeitungstechnik, einer medizinischen Untersuchungsart und/oder einer patientenspezifischen Information automatisch mittels der Anpassungseinheit 11 angepasst wird.

Es kann vorgesehen sein, dass die spektrale Bildverarbeitungstechnik, die medizinische Untersuchungsart und/oder die patientenspezifische Information auch in den Schritt des Ermittelns eingeht.

Weiterhin umfasst das Verfahren S den Schritt des Emittierens. Dabei wird Röntgenstrahlung 4 aufweisend das erste Energiespektrum 9 und aufweisend das zweite Energiespektrum 10 mittels der Röntgenquelleneinheit 3 emittiert.

Das erste Energiespektrum 9 und das zweite Energiespektrum 10 kann zeitgleich mittels einer von der Röntgenquelleneinheit 3 umfassten Röntgenquelle 32 emittiert werden. Dies kann beispielsweise mittels eines Split-Filters, welcher als Vorfilter an der Röntgenquelle 32 angeordnet ist, erreicht werden, und wobei das erste Energiespektrum 9 und das zweite Energiespektrum 10 in zwei unterschiedliche, voneinander abgegrenzte Raumbereiche emittiert werden kann, so dass ein Teil des durch die Röntgenstrahlung belichteten Röntgendetektors 72 mittels des ersten Energiespektrums 9 belichtet wird und ein zweiter Teil des durch die Röntgenstrahlung belichteten Röntgendetektors 72 mittels des zweiten Energiespektrums 10 belichtet wird. Das erste Energiespektrum 9 und das zweite Energiespektrum 10 kann auch zeitlich versetzt mittels einer von der Röntgenquelleneinheit 3 umfassten Röntgenquelle 31 emittiert werden, beispielsweise durch Anwenden der Methode des kV-Switchings, wobei die Röntgenquelle 31 ausgebildet ist in schneller zeitlicher Abfolge entweder das erste Energiespektrum 9 oder das zweite Energiespektrum 10 zu emittieren.

Das erste Energiespektrum 9 und zweite Energiespektrum 10 kann auch von zwei unterschiedlichen Röntgenquellen 33,34, welche von der Röntgenquelleneinheit 3 umfasst sind, emittiert werden, indem eine erste Röntgenquelle 33 der Röntgenquelleneinheit 3 das erste Energiespektrum 9 emittiert und eine zweite Röntgenquelle 34 der Röntgenquelleneinheit 3 das zweite Energiespektrum 10 emittiert. Im Falle eine Dual-Source CT-Geräts umfasst die Röntgenquelleneinheit 3 beispielsweise zwei winkelversetzt angeordnete Röntgenquellen 33,34, welche um eine gemeinsame Rotationsachse 29 um das Untersuchungsobjekt 21 für die Aufnahme von Messsignalen innerhalb unterschiedlicher Winkelsektoren rotierbar angeordnet sind.

Im Schritt des Detektierens S3 wird die emittierte Röntgenstrahlung 4 aufweisend das erste Energiespektrum 9 und aufweisend das zweite Energiespektrum 10 mittels der Detektionseinheit 7 detektiert, wobei zumindest ein erstes Messsignal in Abhängigkeit des ersten Energiebereichs EB1 und des ersten Energiespektrums 9 und zumindest ein zweites Messsignal in Abhängigkeit des zweiten Energiebereichs EB2 und des zweiten Energiespektrums 10 generiert wird.

Anschließend wird in einem Schritt des Erzeugens S4 der spektrale Röntgenbilddatensatz basierend zumindest auf dem generierten ersten und dem generierten zweiten Messsignal mit Hilfe einer spektralen Bildverarbeitungstechnik mittels einer Bildverarbeitungseinheit 13 erzeugt und im Schritt des Ausgebens S5 mittels einer Schnittstelle 15 ausgegeben. Im Gebiet der CT-Bildverarbeitung sind solche spektralen Bildverarbeitungstechniken breit bekannt und sollen daher hier nicht weiter ausgeführt werden.

Der spektrale Röntgenbilddatensatz kann beispielsweise an eine Darstellungseinheit 17 für die Darstellung des spektralen CT-Röntgenbilddatensatzes oder an eine Weiterverarbeitungseinheit für eine Weiterverarbeitung des spektralen CT-Bilddatensatzes ausgegeben werden.

Es kann vorgesehen sein, dass lediglich das erste und das zweite Messsignal in das Erzeugen eingehen. Darüber hinaus können auch weitere Messsignale erzeugt werden. Beispielsweise kann ein drittes Messsignal in Abhängigkeit des ersten Energiespektrums 9 und des zweiten Energiebereichs EB2 oder in Abhängigkeit des ersten Energiespektrums 9 und eines dritten Energiebereichs und ein viertes Messsignal in Abhängigkeit des zweiten Energiespektrums 10 und des ersten Energiebereichs EB1 oder in Abhängigkeit des zweiten Energiespektrums 10 eines vierten Energiebereichs generiert werden und wobei im Schritt des Erzeugens S5 außerdem auch das dritte und vierte Messsignal eingehen.

Dabei kann vorgesehen sein, dass beim Erzeugen des spektralen CT-Bilddatensatzes die generierten Messignale mittels Wichtungsfaktoren gewichtet in den Bilddatensatz eingehen, wobei das erste Messsignal insbesondere höher gewichtet wird als das dritte Messsignal und das zweite Messsignal insbesondere höher gewichtet wird als das vierte Messsignal.

Dafür können insbesondere optimierte Wichtungsfaktoren zum Einsatz kommen, die basierend einem Bildrauschwert des CT-Bilddatensatzes, einem Bildkontrastwert des Röntgenbilddatensatzes oder einem Artefaktwert des CT-Bilddatensatzes optimiert sind. Die optimierten Wichtungsfaktoren für die jeweiligen Messsignale können denjenigen Wichtungsfaktoren entsprechen, welcher zu einer gewünschten bzw. zur Beantwortung einer klinischen Fragestellung notwendigen Bildqualität führen, wenn der CT-Bilddatensatz darauf basieren erzeugt wird.

Das Verfahren kann einen weiteren Schritt S6 des Darstellens des spektralen CT-Bilddatensatzes umfassen, wobei der spektrale CT-Bilddatensatz auf einer Darstellungseinheit 17, beispielsweise einem Display, für einen Anwender dargestellt wird.

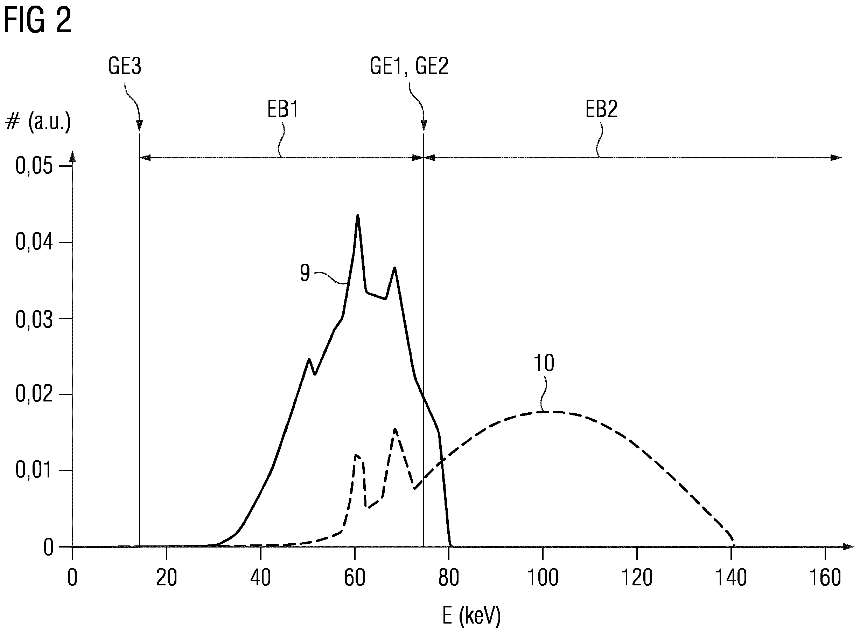

Fig. 2 zeigt zur Veranschaulichung eine mögliche Konfiguration der anpassbaren Energiebereiche EB1, EB2 nach einer Anpassung an das erste Energiespektrum 9 und das zweite Energiespektrum 10.

Ein Energiespektrum 9,10 emittierter Röntgenstrahlung beschreibt im Wesentlichen die Verteilung der emittierten Röntgenphotonen einer Röntgenquelle in Abhängigkeit der Photonenenergie E. In diesem Beispiel basieren beide Energiespektren 9,10 jeweils auf dem Einsatz einer Röntgenröhre mit einer Wolfram-Anode unter Verwendung zweier unterschiedlicher Röhrenspannungen, 80kV und 140kV. Damit wird in diesem Beispiel ein erstes niederenergetischeres Energiespektrum 9 mit einer maximalen Photonenenergie der emittierten Röntgenstrahlung von 80keV und ein zweites, höherenergetischeres Energiespektrum 10 mit einer maximalen Photonenenergie der emittierten Röntgenstrahlung von 140keV mittels der Röntgenquelleneinheit 7 emittiert. Im Falle des zweiten, höherenergetischeren Energiespektrums 10 wurde außerdem die minimale, emittierte Energie, die wesentlich zum spektralen CT-Bilddatensatz beiträgt, mittels eines 0.4mm Zinn-Filters zu höheren Energien zwischen 40keV und 50keV verschoben. In anderen Varianten können auch anderweitige Energiespektren verwendet werden.

In diesem Setup ist der erste Energiebereich EB1 zwischen 15keV und 75keV und ein zweiter Energiebereich EB2 oberhalb von 75keV ausgewiesen. Der erste, obere Grenzenergie GE1 des ersten Energiebereichs EB1 und die zweite, niederenergetische Grenzenergie GE2 des zweiten Energiebereichs EB2 weisen denselben Energiewert von 75keV auf. Der erste Energiebereich EB1 grenzt an den zweiten Energiebereich EB2 an. Des Weiteren wird der erste Energiebereich EB1 von einer unteren Grenzenergie GE3 begrenzt. Es kann auch vorgesehen sein, dass der zweite Energiebereich EB2 von einer oberen Grenzenergie begrenzt ist.

Für die Durchführung des erfindungsgemäßen Verfahrens wird zumindest ein erstes Messsignal in Abhängigkeit des ersten Energiespektrums 9 und des ersten Energiebereichs EB1 und ein zweites Messsignal in Abhängigkeit des zweiten Energiespektrums 10 und des zweiten Energiebereichs EB2 erzeugt.

Eine solche Umsetzung kann beispielsweise mit zwei photonenzählenden Röntgendetektoren 73,74 umgesetzt werden, welche jeweils zumindest zwei Energieschwellen für die Generierung von energieaufgelösten Messsignalen aufweisen, in Kombination mit zwei Röntgenquellen 33,34 in Form eines Dual-Source CT-Geräts.

Das heißt, ausgehend von einem Dual-Source CT-Gerät können die beiden photonenzählenden Röntgendetektoren 73,74 beispielsweise jeweils mit zwei Energiebereichen betrieben werden, wobei für das eine Mess-System bestehend aus einer ersten Röntgenquelle 33 und dem zugeordneten photonenzählenden Röntgendetektor 73, welches das niederenergetische 80kV-Energiespektrum 9 emittiert bzw. detektiert, nur die Daten des niederenergetischen Energietopfes EB1 verwendet werden und für das andere Mess-System, bestehend aus der zweiten Röntgenquelle 34 und dem zugeordneten photonenzählenden Röntgendetektor 74, welches das höherenergetischere 140kV-Energiespektrum 10 emittiert bzw. detektiert, nur die Daten des höherenergetischen Energietopfes EB2 für die Erzeugung verwendet werden.

Abgesehen von der zusätzlichen Zinnfilterung des zweiten Energiespektrums 10 ist eine ähnliche Umsetzung auch mittels eines photonenzählenden Röntgendetektors 31 erreichbar, welcher zumindest zwei Energieschwellen für die Generierung von energieaufgelösten Messsignalen aufweist, in Kombination mit einer Röntgenquelle 31 ausgebildet zu der Methode des kV Switchings, welche zeitlich versetzt entweder das erste oder das zweite Energiespektrum emittiert. Hier wird entsprechend ebenfalls zumindest ein erstes Messsignal in Abhängigkeit des ersten Energiespektrums 9 und des ersten Energiebereichs EB1 und ein zweites Messsignal in Abhängigkeit des zweiten Energiespektrums 10 und des zweiten Energiebereichs EB2 generiert werden.

Darüber hinaus kann außerdem vorgesehen sein, dass ein drittes Messsignal in Abhängigkeit des ersten Energiespektrums 9 und des zweiten Energiebereichs EB2 und ein viertes Messsignal in Abhängigkeit des zweiten Energiespektrums 10 und des ersten Energiebereichs EB1 durch den oder die photonenzählenden Röntgendetektoren generiert werden. Im Schritt des Erzeugens S5 kann dann außerdem auch das dritte und vierte Messsignal eingehen. In diesem komplexeren Fall können die Messsignale der Energietöpfe der photonenzählenden Röntgendetektoren insbesondere gewichtet kombiniert werden, wobei das dritte Messsignal geringer gewichtet wird als das erste Messsignal und das vierte Messsignal geringer gewichtet wird als das zweite Messsignal.

Fig. 2 zeigt eine weitere beispielhafte Konfiguration der Energiebereiche EB1, EB2 nach einer Anpassung an das erste Energiespektrum 9 und das zweite Energiespektrum 10 aus Fig.2, wobei der erste Energiebereich EB1 und der zweite Energiebereich EB2 voneinander beabstandet sind.

So weist die untere, niederenergetische Grenzenergie GE2 des zweiten Energiebereichs EB2 einen höheren Energiewert auf als die obere, höherenergetische Grenzenergie GE1 des ersten Energiebereichs EB1. In diesem Beispiel weist die untere, niederenergetische Grenzenergie GE2 des zweiten Energiebereichs EB2 einen Energiewert von 80keV entsprechend der maximal emittierten Energie des ersten Energiespektrums 9. Die obere, höherenergetische Grenzenergie GE1 des ersten Energiebereichs EB1 weist dagegen einen Energiewert von 70keV auf.

Eine solche Konfiguration kann beispielsweise mittels eines photonenzählenden Röntgendetektors umgesetzt sein, welcher zumindest drei Energieschwellen für die Generierung von energieaufgelösten Messsignalen aufweist und damit einen weiteren Energiebereich energetisch zwischen dem ersten Energiebereich EB1 und dem zweiten Energiebereich EB2 bereitstellen kann. Eine solche Konfiguration kann beispielsweise auch mittels zweier photonenzählender Röntgendetektoren umgesetzt werden, wobei die Energiebereiche des ersten Röntgendetektors und des zweiten Röntgendetektors unterschiedlich angepasst sind. Beispielsweise weist der erste Röntgendetektor einen Energiebereich, entsprechend dem erfindungsgemäßen ersten Energiebereich EB1, zwischen den Grenzenergien GE3 und GE1 und einen Energiebereich energetisch oberhalb der Grenzenergie GE1 auf. Beispielsweise weist der zweite Röntgendetektor einen Energiebereich zwischen der Grenzenergie GE3 und GE2 auf und einen Energiebereich, entsprechend dem erfindungsgemäßen zweiten Energiebereich EB2 oberhalb der Grenzenergie GE2.

Zumindest in Abhängigkeit des erfindungsgemäßen ersten Energiebereichs EB1 und des erfindungsgemäßen zweiten Energiebereich EB2 für das erfindungsgemäße Verfahren das erste Messsignal und das erfindungsgemäße zweite Messsignal erzeugt. Eine solche Konfiguration mit beabstandeten Energiebereichen kann gegebenenfalls zu einer verbesserten Separierung der Messdaten basierend auf den Messsignalen ermöglichen.

Daneben können jedoch neben dem ersten und dem zweiten Messsignal außerdem weitere Messsignale entsprechend den durch die Grenzenergien GE1, GE2, GE3 vorgegebenen Energiebereichen und in Abhängigkeit des ersten Energiespektrums 9 und/oder zweiten Energiespektrums 10 generiert werden. Vorzugweise gehen die weiteren Energiebereiche mit einem geringeren Gewicht in die Erzeugung des spektralen CT-Bilddatensatzes ein, als das erste und das zweite Messsignal. Darüber hinaus sind auch andere Konfigurationen möglich. Beispielsweise sind Ausgestaltungen denkbar, bei denen noch weitere, nicht dargestellte Energiebereiche unterschieden werden.

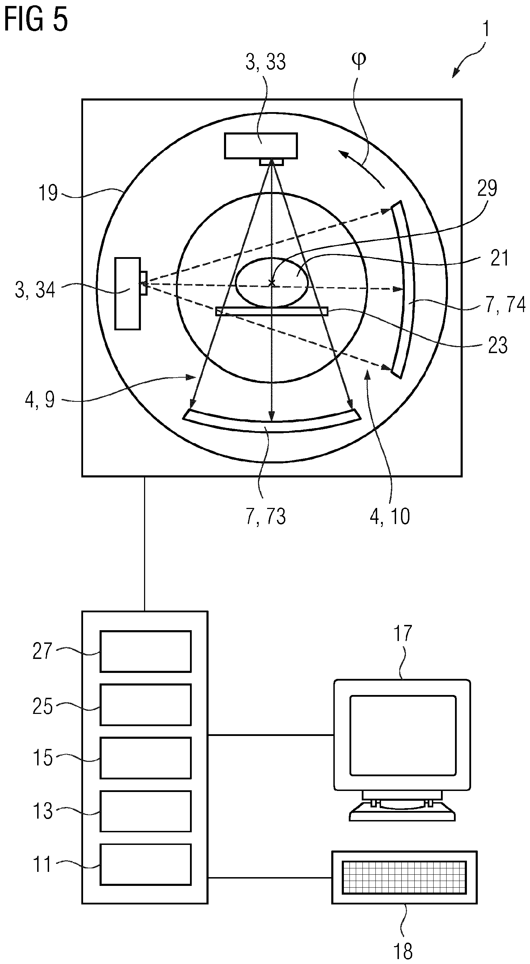

Fig. 5 zeigt eine schematische Darstellung einer Vorrichtung zur Erzeugung eines spektralen Röntgenbilddatensatzes, wobei die Vorrichtung als Computertomographiesystem 1 (CT-System) ausgebildet ist,

Das CT-System 1 umfasst eine Gantry 19 umfassend eine Röntgenquelleneinheit 3 mit einer Röntgenquelle 31 und in Gegenüberstellung eine Detektionseinheit 7 mit einem photonenzählenden Röntgendetektor 71. Ein Untersuchungsobjekt 21 ist auf einer Liege 23 gelagert und ist entlang der Rotationsachse 29 durch die Gantryöffnung für die Aufnahme der Projektionsmessdaten bewegbar. Zur Aufnahme räumlich dreidimensionaler Bilddaten rotiert die Röntgenquelleneinheit 3 sowie die Detektionseinheit 7 in phi-Richtung um das zu untersuchende Objekt 21, wobei Röntgenstrahlung 9,10 von der Röntgenquelle 31 emittiert und nach Durchgang durch das Objekt 21 vom Röntgendetektor 71 detektiert wird. Während der Rotationsbewegung werden innerhalb eines Winkelsektors jeweils Messsignale generiert.

Die Röntgenquelleneinheit 3 ist insbesondere ausgebildet, Röntgenstrahlung 4 aufweisend ein erstes Energiespektrum 9 und Röntgenstrahlung 4 aufweisend ein zweites, vom ersten verschiedenes Energiespektrum 10 zu emittieren. Dazu weist in diesem Beispiel die Röntgenquelleneinrichtung 3 eine Röntgenquelle 31, insbesondere eine Röntgenröhre auf, welche ausgebildet ist mit der Methode des "kV Switchings" ("kV Umschaltens") betrieben zu werden. Das heißt, die Röntgenquelle 31 emittiert während der Rotation um das Untersuchungsobjekt selektiv abwechselnd in kurzer zeitlicher Abfolge entweder ein erstes Energiespektrum 9 oder ein zweites Energiespektrum 10, wobei das zweite Energiespektrum 10 sich vom ersten Energiespektrum 9 unterscheidet. In der Regel wird die Röntgenquelle mit zwei unterschiedlichen Röhrenspannungen, beispielsweise einer ersten, niedrigeren Röhrenspannung und einer zweiten, höhere Röhrenspannung betrieben, so dass abwechselnd Röntgenstrahlung mit dem ersten, in diesem Fall niederenergetischen Energiespektrum und Röntgenstrahlung mit dem zweiten, höherenergetischen Energiespektrum emittiert wird.

Die Detektionseinheit 7 weist einen photonenzählenden Röntgendetektor 71 auf, welcher der Röntgenquelle 31 gegenüberliegend positioniert ist. Der photonenzählende Röntgendetektor 71 der Detektionseinheit 7 wird während der Rohdatenaufnahme dementsprechend abwechselnd entweder mit dem ersten Energiespektrum 9 oder mit dem zweiten Energiespektrum 10 belichtet.

Die Detektionseinheit 7 ist ausgebildet basierend auf detektierter Röntgenstrahlung 4 Messsignale zu generieren, welche zumindest in einen ersten anpassbaren Energiebereich EB1 und einen zweiten, anpassbaren Energiebereich EB2 aufgelöst sind.

Darüber hinaus kann der photonenzählende Röntgendetektor auch ausgebildet sein, detektierte Röntgenstrahlung in weitere anpassbare Energiebereiche aufzulösen.

Die Vorrichtung weist außerdem eine Anpassungseinheit 11 auf, welche ausgebildet ist den ersten Energiebereich EB1 und den zweiten Energiebereich EB2 zumindest in Abhängigkeit des ersten Energiespektrums 9 und des zweiten Energiespektrums 10 anzupassen, wobei jeweils zumindest eine Grenzenergie GE1, GE2 eines jeweiligen Energiebereichs EB1, EB2 angepasst wird. Das Anpassen in Abhängigkeit des ersten Energiespektrums und des zweiten Energiespektrums kann umfassen, dass die jeweilige zumindest eine Grenzenergie in Abhängigkeit des ersten und des zweiten Energiespektrums gewählt und angewendet wird.

In einer vorteilhaften Ausgestaltung der Erfindung kann vorgesehen sein, dass die Anpassungseinheit außerdem ausgebildet ist, die jeweilige zumindest eine Grenzenergie des ersten Energiebereichs und des zweiten Energiebereichs in Abhängigkeit der spektralen Bildverarbeitungstechnik, einer medizinischen Untersuchungsart und/oder einer patientenspezifischen Information automatisch mittels der Anpassungseinheit anzupassen.

Die Vorrichtung umfasst außerdem eine Bildverarbeitungseinheit 13, ausgebildet den spektralen CT-Bilddatensatz mittels einer spektralen Bildverarbeitungstechnik basierend auf den generierten Messignalen zu erzeugen, wobei zumindest das erste, generierte Messignal in Abhängigkeit des ersten Energiebereichs EB1 und des ersten Energiespektrums 9 und das zweite, generierte Messignal in Abhängigkeit des zweiten Energiebereich EB2 und des zweiten Energiespektrums 10 in das Erzeugen miteingehen.

Stehen weitere Messsignale zur Verfügung können entsprechen auch die weiteren Messsignale in die Erzeugung miteingehen. Insbesondere können die generierten Messsignale dabei mittels Wichtungsfaktoren gewichtet in die Erzeugung des CT-Bilddatensatzes eingehen. Dabei können insbesondere optimierte Gewichtungsfaktoren zum Einsatz kommen, die basierend einem Bildrauschwert des CT-Bilddatensatzes, einem Bildkontrastwert des CT-Bilddatensatzes oder einem Artefaktwert des CT-Bilddatensatzes optimiert sind.

Des Weiteren umfasst die Vorrichtung eine Schnittstelle 15, ausgebildet den spektralen Röntgenbilddatensatz auszugeben. Insbesondere wird in diesem Beispiel der spektrale CT-Bilddatensatz an eine Ausgabeeinheit 17 in Form einer Darstellungseinheit ausgegeben. Mittels der Ausgabeeinheit 17 in Form der Darstellungseinheit kann der spektrale CT-Bilddatensatzes für einen Bediener angezeigt werden.

Die Vorrichtung weist außerdem eine Optimierungseinheit 25 auf, welche ausgebildet ist einen optimierten Energiewert für die jeweilige zumindest eine Grenzenergie des ersten Energiebereichs und des zweiten Energiebereichs zumindest basierend auf dem ersten und dem zweiten Energiespektrum zu ermitteln.

Erfindungsgemäß wird dabei der optimierte Energiewert anhand zumindest eines Kriteriums der folgenden Liste optimiert
- Ein Bildrauschwert des CT-Bilddatensatzes
- Einen Bildkontrastwert des CT-Bilddatensatzes
- Ein Artefaktwert des CT-Bilddatensatzes
- Ein Wert des spektralen Überlapp.

Dabei kann auch die spektralen Bildverarbeitungstechnik, einer medizinischen Untersuchungsart und/oder patientenspezifische Informationen miteingehen.

Die Optimierungseinheit kann ausgebildet sein den optimierten Energiewert basierend auf einem maschinellen Lernverfahrens, einer arithmetischen Funktion bzw. Berechnung oder auch anderweitig zu ermitteln.

Die Anpassungseinheit 11, die Bildverarbeitungseinheit 13 und ggf. die Optimierungseinheit 25 kann insbesondere in Form eines Computers, eines Mikrocontrollers oder eines integrierten Schaltkreises umgesetzt sein. Die Anpassungseinheit 11, die Bildverarbeitungseinheit 13 und ggf. die Optimierungseinheit 25 kann Hardware-Elemente oder Software-Elemente aufweisen, beispielsweise einen Mikroprozessor oder ein sogenanntes FPGA (englisches Akronym für "Field Programmable Gate Array"). Es kann sich auch um einen realen oder virtuellen Verbund von Computern handeln (ein englischer Fachbegriff für einen realen Verbund ist "Cluster", ein englischer Fachbegriff für einen virtuellen Verbund ist "Cloud").

Die Vorrichtung kann außerdem eine Speichereinheit 27 umfassen. Diese kann als nicht dauerhafte Arbeitsspeicher (Random Access Memory, kurz RAM) oder als dauerhafter Massen-speicher (Festplatte, USB-Stick, SD-Karte, Solid State Disk) realisiert sein. Bei einer Schnittstelle 15 kann es sich um eine Hardware- oder Softwareschnittstelle handeln (beispielsweise PCI-Bus, USB oder Firewire).

Die Speichereinheit kann dafür vorgesehen sein bereits im Vorfeld mittels der Optimierungseinheit ermittelte optimierte Energiewerte für eine Vielzahl von ersten und zweiten Energiespektren in Abhängigkeit des ersten und zweiten Energiespektrums, der spektralen Bildverarbeitungstechnik, der medizinischen Untersuchungsart und/oder patientenspezifischer Informationen oder auch anderer Parameter, etwa in Form einer Datenbank, durch die Anpassungseinheit abrufbar vorzuhalten. Die Speichereinheit kann dafür vorgesehen sein trainierte bzw. angepasste Funktionen abrufbar bereitzustellen, welche in Abhängigkeit des ersten und zweiten Energiespektrum, der spektralen Bildverarbeitungstechnik, einer medizinischen Untersuchungsart und/oder einer patientenspezifische Information oder auch andere Parameter als Eingangsparameter den optimierten Energiewert ermitteln können. Für den Schritt des Ermittelns können diese dann abgerufen werden.

Optimalerweise weist die Vorrichtung weiterhin wenigstens eine Eingabeeinheit 18 auf. Eine Eingabeeinheit 18 ermöglicht beispielsweise die manuelle Interaktion eines Anwenders, beispielsweise, das Starten oder Stoppen des erfindungsgemäßen Verfahrens. Ebenso kann die Auswahl oder Bestätigung einer Bildverarbeitungstechnik oder einer Röntgenanwendung, oder die Eingabe des zu untersuchenden Untersuchungsobjekts oder patientenspezifischer Information für einen Bediener ermöglicht werden.

Fig. 5 zeigt eine alternative Ausführung einer erfindungsgemäßen Vorrichtung. In diesem Beispiel weist die Detektionseinheit 7 einen ersten photonenzählenden Röntgendetektor 73 und einen winkelversetzt dazu angeordneten, zweiten photonenzählenden Röntgendetektor 74 auf. Die Röntgenquelleneinheit 3 weist jeweils in Gegenüberstellung dazu eine erste Röntgenquelle 33 auf, ausgebildet das erste Energiespektrum 9 zu emittieren, und weist eine zweite Röntgenquelle 34 auf, ausgebildet das zweite Energiespektrum 10 zu emittiert. Dieses Setup entspricht einem bereits erwähnten Dual Source CT-System.

In dieser Konfiguration ist insbesondere der erste photonenzählenden Röntgendetektor 73 ausgebildet, zumindest den ersten Energiebereich EB1 für energieaufgelöste Messsignale bereitzustellen und zumindest das erste Messignal in Abhängigkeit des ersten Energiebereichs EB1 und des ersten Energiespektrums 9 zu generieren. Der zweite photonenzählenden Röntgendetektor 74 ist dann insbesondere ausgebildet, zumindest den zweiten Energiebereich EB2 für energieaufgelöste Messsignale bereitzustellen und zumindest das zweite Messignal in Abhängigkeit des zweiten Energiebereichs EB2 und des zweiten Energiespektrums 10 zu generieren. Darüber hinaus können die jeweiligen photonenzählenden Röntgendetektoren 73,74 der Detektionseinheit 7 auch noch weitere Energiebereiche für die Generierung von Messsignalen zur Verfügung stellen, welche in das Erzeugen des CT-Bilddatensatzes miteingehen können.

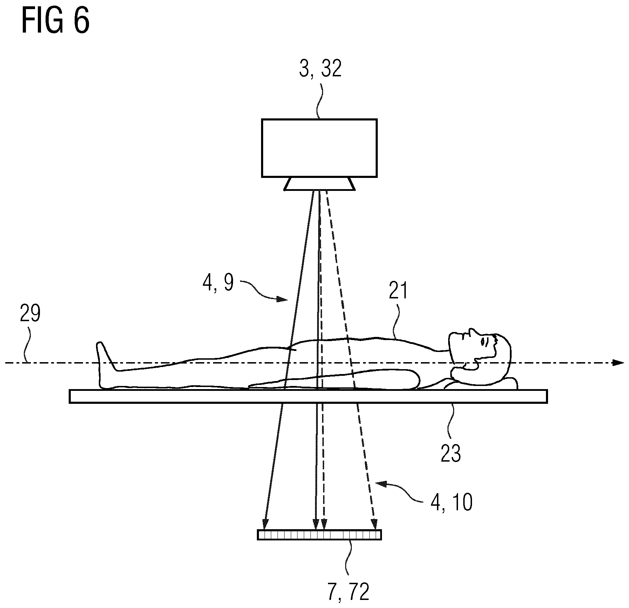

Fig. 6 zeigt schematisch eine Konfiguration aus Röntgenquelleneinheit 3, aufweisend eine Röntgenquelle 32, und Detektionseinheit 7, aufweisend einen photonenzählenden Röntgendetektor 72, wie sie in einer weiteren Variante der erfindungsgemäßen Vorrichtung zum Einsatz kommen kann. In dieser Konfiguration ist die Röntgenquelle 32 mit einem Split-Filter ausgebildet, welcher einen entlang der Rotationsachse 29, auch Patientenachse genannt, geteilten Vorfilter darstellt, sodass in unterschiedliche Raumbereiche gleichzeitig zwei voneinander verschiedene Energiespektren, d.h. das erste Energiespektrum 9 und das zweite Energiespektrum 10, mittels der Röntgenquelle emittiert werden kann. Der photonenzählende Röntgendetektor 72 weist eine, hier lediglich schematisch angedeutete Mehrzahl an Detektionselementen auf, wobei eine erste Teilzahl der Mehrzahl an Detektionselementen mittels der Röntgenquelleneinheit 3 mit dem ersten Energiespektrum 9 belichtet wird und eine zweite Teilzahl der Mehrzahl mit dem zweiten Energiespektrum 10 belichtet wird, so dass mittels der ersten Teilzahl der Mehrzahl an Detektionselementen das erste Messignal und mittels der zweiten Teilzahl der Mehrzahl an Detektionselementen das zweite Messignal generiert und für das Erzeugen des spektralen CT-Bilddatensatzes bereitgestellt werden kann.

Der Gegenstand der Erfindung ist nicht auf die vorstehend beschriebenen Ausführungsbeispiele beschränkt. Vielmehr können weitere Ausführungsformen der Erfindung von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden.

## Patentansprüche

1. Verfahren zur Erzeugung eines spektralen Computertomographie-Bilddatensatzes mittels einer Detektionseinheit (7), aufweisend zumindest einen photonenzählenden Röntgendetektor (71,72,73,74) und ausgebildet detektierte Röntgenstrahlung (4) in Messsignale umzuwandeln, welche zumindest in einen ersten, anpassbaren Energiebereich (EB1) und einen zweiten, anpassbaren Energiebereich (EB2) aufgelöst sind, und mittels einer Röntgenquelleneinheit (3), ausgebildet Röntgenstrahlung (4) aufweisend ein erstes Energiespektrum (9) und aufweisend ein zweites, vom ersten verschiedenes Energiespektrum (10) zu emittieren, umfassend die Schritte
- Anpassen (S1) des ersten Energiebereichs (EB1) und des zweiten Energiebereichs (EB2) in Abhängigkeit des ersten Energiespektrums (9) und des zweiten Energiespektrums (10) mittels einer Anpassungseinheit (11), wobei jeweils zumindest eine Grenzenergie (GE1, GE2) eines jeweiligen Energiebereichs (EB1, EB2) angepasst wird,
- Emittieren (S2) von Röntgenstrahlung (4) aufweisend das erste Energiespektrum (9) und aufweisend das zweite Energiespektrum (10) mittels der Röntgenquelleneinheit (3),
- Detektieren (S3) der emittierten Röntgenstrahlung (4) aufweisend das erste Energiespektrum (9) und aufweisend das zweite Energiespektrum (10) mittels der Detektionseinheit (7), wobei zumindest ein erstes Messsignal in Abhängigkeit des ersten Energiebereichs (EB1) und des ersten Energiespektrums (9) und zumindest ein zweites Messsignal in Abhängigkeit des zweiten Energiebereichs (EB2) und des zweiten Energiespektrums (10) generiert wird,
- Erzeugen (S4) des spektralen Computertomographie-Bilddatensatzes basierend zumindest auf dem generierten ersten und dem generierten zweiten Messsignal mit Hilfe einer spektralen Bildverarbeitungstechnik mittels einer Bildverarbeitungseinheit (13),
- Ausgeben (S5) des spektralen Computertomographie-Bilddatensatzes mittels einer Schnittstelle (15), **dadurch gekennzeichnet, dass** das Verfahren weiterhin umfasst
- Ermitteln (S0) eines optimierten Energiewerts für die jeweilige zumindest eine Grenzenergie (GE1, GE2) des ersten Energiebereichs (EB1) und des zweiten Energiebereichs (EB2) zumindest basierend auf dem ersten Energiespektrum (9) und dem zweiten Energiespektrum (10) mittels einer Optimierungseinheit (25), wobei außerdem zumindest ein Kriterium der folgenden Liste optimiert wird
• Ein Bildrauschwert des Computertomographie-Bilddatensatzes,
• Ein Bildkontrastwert des Computertomographie-Bilddatensatzes,
• Ein Materialkontrastwert des Computertomographie-Bilddatensatzes,
• Ein Artefaktwert des Computertomographie-Bilddatensatzes,
• Ein Wert des spektralen Überlapp zwischen dem ersten Energiespektrum (9) und dem zweiten Energiebereich (EB2) und/oder dem zweiten Energiespektrum (10) und dem ersten Energiebereich (EB1), und
wobei das Anpassen (S1) auf dem optimierten Energiewert für die jeweilige zumindest eine Grenzenergie (GE1, GE2) basiert.

2. Verfahren nach Anspruch 1, wobei im Schritt des Anpassens (S1) die jeweilige zumindest eine Grenzenergie (GE1, GE2) des ersten Energiebereichs (EB1) und des zweiten Energiebereichs (EB2) außerdem in Abhängigkeit der spektralen Bildverarbeitungstechnik, einer medizinischen Untersuchungsart und/oder einer patientenspezifischen Information automatisch mittels der Anpassungseinheit (11) angepasst wird.

3. Verfahren nach einem der vorstehenden Ansprüche, wobei das Ermitteln (S0) das Anwenden eines maschinellen Lernverfahrens umfasst.

4. Verfahren nach einem der vorstehenden Ansprüche, wobei der erste Energiebereich (EB1) an den zweiten Energiebereich (EB2) angrenzt.

5. Verfahren nach einem der Ansprüche 1 bis 3, wobei der erste Energiebereich (EB1) und der zweite Energiebereich (EB2) voneinander beabstandet sind.

6. Verfahren nach einem der vorstehenden Ansprüche, wobei außerdem ein drittes Messsignal in Abhängigkeit des ersten Energiespektrums (9) und des zweiten Energiebereichs (EB2) oder in Abhängigkeit des ersten Energiespektrums (9) und eines dritten Energiebereichs (EB3) und ein viertes Messsignal in Abhängigkeit des zweiten Energiespektrums (10) und des ersten Energiebereichs (EB1) oder in Abhängigkeit des zweiten Energiespektrums (10) eines vierten Energiebereichs (EB4) generiert werden und wobei im Schritt des Erzeugens (S5) außerdem auch das dritte und vierte Messsignal eingehen.

7. Verfahren nach Anspruch 6, wobei beim Erzeugen (S4) des spektralen Computertomographie-Bilddatensatzes die generierten Messignale mittels optimierter Wichtungsfaktoren gewichtet in den Bilddatensatz eingehen und wobei das erste Messsignal höher gewichtet wird als das dritte Messsignal und das zweite Messsignal höher gewichtet wird als das vierte Messsignal.

8. Verfahren nach Anspruch 7, wobei beim Erzeugen (S4) des spektralen Computertomographie-Bilddatensatzes die generierten Messignale mittels optimierter Wichtungsfaktoren gewichtet in den Bilddatensatz eingehen und wobei die Wichtungsfaktoren basierend auf zumindest einem Kriterium der folgenden Liste optimiert sind
• Ein Bildrauschwert des Computertomographie-Bilddatensatzes,
• Einen Bildkontrastwert des Computertomographie-Bilddatensatzes,
• Ein Materialkontrastwert des Computertomographie-Bilddatensatzes,
• Ein Artefaktwert des Computertomographie-Bilddatensatzes.

9. Verfahren nach einem der vorstehenden Ansprüche, wobei im Schritt des Emittierens (S2) selektiv abwechselnd entweder das erste Energiespektrum (9) oder das zweite Energiespektrum (10) emittiert wird.

10. Verfahren nach einem der Ansprüche 1 bis 8, wobei die Detektionseinheit (7) einen photonenzählenden Röntgendetektor (72) mit einer Mehrzahl an Detektionselementen aufweist, und wobei eine erste Teilzahl der Mehrzahl an Detektionselementen mittels der Röntgenquelleneinheit (3) mit dem ersten Energiespektrum (9) belichtet wird und eine zweite Teilzahl der Mehrzahl mit dem zweiten Energiespektrum (10) belichtet wird und wobei mittels der ersten Teilzahl der Mehrzahl an Detektionselementen das erste Messignal und mittels der zweiten Teilzahl der Mehrzahl an Detektionselementen das zweite Messignal generiert wird.

11. Verfahren nach einem der Ansprüche 1 bis 8, wobei die Detektionseinheit (7) einen ersten photonenzählenden Röntgendetektor (73) und einen winkelversetzt dazu angeordneten, zweiten photonenzählenden Röntgendetektor (74) aufweist, und die Röntgenquelleneinheit (3) jeweils in Gegenüberstellung dazu eine erste Röntgenquelle (33), welche das erste Energiespektrum (9) emittiert, und eine zweite Röntgenquelle (34), welche das zweite Energiespektrum (10) emittiert, aufweist, und wobei mittels des ersten Röntgendetektors (73) das erste Messsignal und mittels des zweiten Röntgendetektors (74) das zweite Messignal generiert wird.

12. Vorrichtung zur Erzeugung eines spektralen Computertomographie-Bilddatensatzes umfassend
- Eine Röntgenquelleneinheit (3), ausgebildet Röntgenstrahlung (4) aufweisend ein erstes Energiespektrum (9) und Röntgenstrahlung (4) aufweisend ein zweites, vom ersten verschiedenes Energiespektrum (10) zu emittieren,
- Einer Detektionseinheit (7), aufweisend zumindest einen photonenzählenden Röntgendetektor (71,72,73,74) und ausgebildet basierend auf detektierter Röntgenstrahlung (4) Messsignale zu generieren, welche zumindest in einen ersten anpassbaren Energiebereich (EB1) und einen zweiten, anpassbaren Energiebereich (EB2) aufgelöst sind,
- Eine Anpassungseinheit (11), ausgebildet den ersten Energiebereich (EB1) und den zweiten Energiebereich (EB2) in Abhängigkeit des ersten Energiespektrums (9) und des zweiten Energiespektrums (10) anzupassen, wobei jeweils zumindest eine Grenzenergie (GE1, GE2) eines jeweiligen Energiebereichs (EB1, EB2) angepasst wird,
- Eine Bildverarbeitungseinheit (13), ausgebildet den spektralen Computertomographie-Bilddatensatzes mittels einer spektralen Bildverarbeitungstechnik basierend auf den generierten Messignalen zu erzeugen, wobei zumindest ein erstes, generiertes Messignal in Abhängigkeit des ersten Energiebereichs (EB1) und des ersten Energiespektrums (9) und ein zweites, generiertes Messignal in Abhängigkeit des zweiten Energiebereich (EB2) und des zweiten Energiespektrums (10) in das Erzeugen miteingehen,
- Eine Schnittstelle (15) zur Ausgabe des spektralen Computertomographie-Bilddatensatzes,
**dadurch gekennzeichnet, dass** die Vorrichtung weiterhin umfasst
- Eine Optimierungseinheit (25), ausgebildet zum Ermitteln (S0) eines optimierten Energiewerts für die jeweilige zumindest eine Grenzenergie (GE1, GE2) des ersten Energiebereichs (EB1) und des zweiten Energiebereichs (EB2) zumindest basierend auf dem ersten Energiespektrum (9) und dem zweiten Energiespektrum (10) mittels einer Optimierungseinheit (25), wobei außerdem zumindest ein Kriterium der folgenden Liste optimiert wird
• Ein Bildrauschwert des Computertomographie-Bilddatensatzes,
• Ein Bildkontrastwert des Computertomographie-Bilddatensatzes,
• Ein Materialkontrastwert des Computertomographie-Bilddatensatzes,
• Ein Artefaktwert des Computertomographie-Bilddatensatzes,
• Ein Wert des spektralen Überlapp zwischen dem ersten Energiespektrum (9) und dem zweiten Energiebereich (EB2) und/oder dem zweiten Energiespektrum (10) und dem ersten Energiebereich (EB1),
wobei das Anpassen (S1) des ersten Energiebereichs (EB1) und des zweiten Energiebereichs (EB2)auf dem optimierten Energiewert für die jeweilige zumindest eine Grenzenergie (GE1, GE2) basiert.

13. Vorrichtung nach Anspruch 12, außerdem ausgebildet ein Verfahren nach einem der Ansprüche 2 bis 11 auszuführen.

14. Computertomographiesystem (1) umfassend eine Vorrichtung nach einem der Ansprüche 12 oder 13.

## Claims

1. Method for producing a spectral computed tomography image data set by means of a detection unit (7), having at least one photon-counting X-ray detector (71, 72, 73, 74) and configured to convert detected X-rays (4) into measurement signals which are resolved at least into a first adaptable energy range (EB1) and a second adaptable energy range (EB2), and by means of an X-ray source unit (3), configured to emit X-rays (4) having a first energy spectrum (9) and having a second energy spectrum (10) which differs from the first, comprising the steps of:
- adapting (S1) the first energy range (EB1) and the second energy range (EB2) as a function of the first energy spectrum (9) and the second energy spectrum (10) by means of an adaptation unit (11), wherein in each case at least one limiting energy (GE1, GE2) of a respective energy range (EB1, EB2) is adapted,
- emitting (S2) X-rays (4) having the first energy spectrum (9) and having the second energy spectrum (10) by means of the X-ray source unit (3),
- detecting (S3) the emitted X-rays (4) having the first energy spectrum (9) and having the second energy spectrum (10) by means of the detection unit (7), wherein at least one first measurement signal is generated as a function of the first energy range (EB1) and the first energy spectrum (9) and at least one second measurement signal as a function of the second energy range (EB2) and the second energy spectrum (10),
- producing (S4) the spectral computed tomography image data set at least on the basis of the generated first and the generated second measurement signals with the assistance of a spectral image processing technique by means of an image processing unit (13),
- outputting (S5) the spectral computed tomography image data set by means of an interface (15), **characterised in that** the method further comprises
- determining (S0) an optimised energy value for the respective at least one limiting energy (GE1, GE2) of the first energy range (EB1) and the second energy range (EB2) at least on the basis of the first energy spectrum (9) and the second energy spectrum (10) by means of an optimisation unit (25), wherein moreover at least one criterion from the following list is optimised:
• an image noise value of the computed tomography image data set,
• an image contrast value of the computed tomography image data set,
• a material contrast value of the computed tomography image data set,
• an artifact value of the computed tomography image data set,
• a spectral overlap value between the first energy spectrum (9) and the second energy range (EB2) and/or the second energy spectrum (10) and the first energy range (EB1), and
wherein the adapting (S1) is based on the optimised energy value for the respective at least one limiting energy (GE1, GE2) .

2. Method according to claim 1, wherein in the adaptation step (S1) the respective at least one limiting energy (GE1, GE2) of the first energy range (EB1) and the second energy range (EB2) is moreover automatically adapted as a function of the spectral image processing technique, a type of medical examination and/or an item of patient-specific information by means of the adaptation unit (11).

3. Method according to one of the preceding claims, wherein the determination (S0) comprises the application of a machine learning method.

4. Method according to one of the preceding claims, wherein the first energy range (EB1) adjoins the second energy range (EB2) .

5. Method according to one of claims 1 to 3, wherein the first energy range (EB1) and the second energy range (EB2) are spaced apart from one another.

6. Method according to one of the preceding claims, wherein moreover a third measurement signal may be generated as a function of the first energy spectrum (9) and the second energy range (EB2) or as a function of the first energy spectrum (9) and a third energy range (EB3) and a fourth measurement signal as a function of the second energy spectrum (10) and the first energy range (EB1) or as a function of the second energy spectrum (10) and a fourth energy range (EB4), and wherein the third and fourth measurement signals are moreover also included in the production step (S5).

7. Method according to claim 6, wherein, on production (S4) of the spectral computed tomography image data set, the generated measurement signals are included in the image data set in a manner weighted by means of optimised weighting factors and wherein the first measurement signal is more heavily weighted than the third measurement signal and the second measurement signal more heavily weighted than the fourth measurement signal.

8. Method according to claim 7, wherein, on production (S4) of the spectral computed tomography image data set, the generated measurement signals are included in the image data set in a manner weighted by means of optimised weighting factors and wherein the weighting factors are optimised on the basis of at least one criterion from the following list:
• an image noise value of the computed tomography image data set,
• an image contrast value of the computed tomography image data set,
• a material contrast value of the computed tomography image data set,
• an artifact value of the computed tomography image data set.

9. Method according to one of the preceding claims, wherein, in the emission step (S2), either the first energy spectrum (9) or the second energy spectrum (10) is selectively alternately emitted.

10. Method according to one of claims 1 to 8, wherein the detection unit (7) has a photon-counting X-ray detector (72) with a plurality of detection elements, and wherein a first subset of the plurality of detection elements is illuminated with the first energy spectrum (9) by means of the X-ray source unit (3) and a second subset of the plurality is illuminated with the second energy spectrum (10) and wherein the first measurement signal is generated by means of the first subset of the plurality of detection elements and the second measurement signal by means of the second subset of the plurality of detection elements.

11. Method according to one of claims 1 to 8, wherein the detection unit (7) has a first photon-counting X-ray detector (73) and, arranged at an angular offset thereto, a second photon-counting X-ray detector (74), and the X-ray source unit (3) in each case has opposite thereto a first X-ray source (33) which emits the first energy spectrum (9) and a second X-ray source (34) which emits the second energy spectrum (10), and wherein the first measurement signal is generated by means of the first X-ray detector (73) and the second measurement signal by means of the second X-ray detector (74).

12. An apparatus for producing a spectral computed tomography image data set comprising:
- an X-ray source unit (3) configured to emit X-rays (4) having a first energy spectrum (9) and X-rays (4) having a second energy spectrum (10) which differs from the first,
- a detection unit (7) having at least one photon-counting X-ray detector (71, 72, 73, 74) and configured to generate measurement signals on the basis of detected X-rays (4), which measurement signals are resolved at least into a first adaptable energy range (EB1) and a second adaptable energy range (EB2),
- an adaptation unit (11) which is configured to adapt the first energy range (EB1) and the second energy range (EB2) as a function of the first energy spectrum (9) and the second energy spectrum (10), wherein in each case at least one limiting energy (GE1, GE2) of a respective energy range (EB1, EB2) is adapted,
- an image processing unit (13) configured to produce the spectral computed tomography image data set by means of a spectral image processing technique on the basis of the generated measurement signals, wherein at least one first generated measurement signal is included in the production as a function of the first energy range (EB1) and the first energy spectrum (9) and a second generated measurement signal as a function of the second energy range (EB2) and the second energy spectrum (10),
- an interface (15) for outputting the spectral computed tomography image data set, **characterised in that** the apparatus further comprises
- an optimisation unit (25), configured for determining (S0) an optimised energy value for the respective at least one limiting energy (GE1, GE2) of the first energy range (EB1) and the second energy range (EB2) at least on the basis of the first energy spectrum (9) and the second energy spectrum (10) by means of an optimisation unit (25), wherein moreover at least one criterion from the following list is optimised:
• an image noise value of the computed tomography image data set,
• an image contrast value of the computed tomography image data set,
• a material contrast value of the computed tomography image data set,
• an artifact value of the computed tomography image data set,
• a spectral overlap value between the first energy spectrum (9) and the second energy range (EB2) and/or the second energy spectrum (10) and the first energy range (EB1),
wherein adaptation (S1) of the first energy range (EB1) and the second energy range (EB2) is based on the optimised energy value for the respective at least one limiting energy (GE1, GE2).

13. Apparatus according to claim 12, moreover configured to carry out a method according to one of claims 2 to 11.

14. Computed tomography system (1) comprising an apparatus according to one of claims 12 or 13.

## Revendications

1. Procédé de production d'un ensemble spectral de données d'image de tomodensitométrie assistée par ordinateur au moyen d'une unité (7) de détection, comportant au moins un détecteur (71, 72, 73, 74) de rayons X contenant des photons et constitué pour transformer du rayonnement (4) X détecté en signaux de mesure, qui sont résolus au moins en une première plage (EB1) d'énergie adaptable et en une deuxième plage (EB2) d'énergie adaptable, et pour émettre au moyen d'une unité (3) de source de rayons X du rayonnement (4) X comportant un premier spectre (9) d'énergie et comportant un deuxième spectre (10) d'énergie différent du premier, comprenant les stades
- adaptation (S1) de la première plage (EB1) d'énergie et de la deuxième plage (EB2) d'énergie en fonction du premier spectre (9) d'énergie et du deuxième spectre (10) d'énergie au moyen d'une unité (11) d'adaptation, dans lequel on adapte respectivement au moins une énergie (GE1, GE2) limite d'une plage (EB1, EB2) d'énergie respective,
- émission (S2) de rayonnement (4) X comportant le premier spectre (9) d'énergie et comportant le deuxième spectre (10) d'énergie au moyen de l'unité (3) de source de rayons X,
- détection (S3) du rayonnement (4) X émis comportant le premier spectre (9) d'énergie et comportant le deuxième spectre (10) d'énergie au moyen de l'unité (7) de détection, dans lequel on crée au moins un premier signal de mesure en fonction de la première plage (EB1) et du premier spectre (9) d'énergie, et au moins un deuxième signal de mesure en fonction de la deuxième plage (EB2) d'énergie et du deuxième spectre (10) d'énergie,
- production (S4), à l'aide d'une technique spectrale de traitement d'image au moyen d'une unité (13) de traitement d'image, de l'ensemble spectral de données d'image de tomodensitométrie assistée par ordinateur, sur la base d'au moins le premier signal de mesure créé et du deuxième signal de mesure créé,
- émission (S5) de l'ensemble spectral de données d'image de tomodensitométrie assistée par ordinateur au moyen d'une interface (15),
**caractérisé en ce que** le procédé comprend en outre
- détermination (S0) d'une valeur d'énergie optimisée pour la au moins une énergie (GE1, GE2) limite respective de la première plage (EB1) et de la deuxième plage (EB2) d'énergie, au moins sur la base du premier spectre (9) d'énergie et du deuxième spectre (10) d'énergie, au moyen d'une unité (25) d'optimisation, dans lequel en outre on optimise au moins un critère de la liste suivante
• une valeur de bruit d'image de l'ensemble de données d'image de tomodensitométrie assistée par ordinateur,
• une valeur de contraste d'image de l'ensemble de données d'image de tomodensitométrie assistée par ordinateur,
• une valeur de contraste de matériau de l'ensemble de données d'image de tomodensitométrie assistée par ordinateur,
• une valeur d'artéfact de l'ensemble de données d'image de tomodensitométrie assistée par ordinateur,
• une valeur du chevauchement spectral entre le premier spectre (9) d'énergie et la deuxième plage (EB2) d'énergie et/ou entre le deuxième spectre (10) d'énergie et la première plage (EB1) d'énergie, et
dans lequel l'adaptation (S1) repose sur la valeur d'énergie optimisée de la au moins une énergie (GE1, GE2) limite respective.

2. Procédé suivant la revendication 1, dans lequel, dans le stade de l'adaptation (S1), on adapte automatiquement au moyen de l'unité (11) d'adaptation la au moins une énergie (GE1, GE2) limite respective de la première plage (EB1) d'énergie et de la deuxième plage (EB2) d'énergie en outre en fonction de la technique spectrale de traitement d'image, d'un type d'examen médical et/ou d'une information spécifique au patient.

3. Procédé suivant l'une des revendications précédentes, dans lequel la détermination (S0) comprend l'utilisation d'un procédé d'apprentissage automatique.

4. Procédé suivant l'une des revendications précédentes, dans lequel la première plage (EB1) d'énergie est voisine de la deuxième plage (EB2) d'énergie.

5. Procédé suivant l'une des revendications 1 à 3, dans lequel la première plage (EB1) d'énergie et la deuxième plage (EB2) d'énergie sont à distance l'une de l'autre.

6. Procédé suivant l'une des revendications précédentes, dans lequel on crée en outre un troisième signal de mesure, en fonction du premier spectre (9) d'énergie et de la deuxième plage (EB2) d'énergie ou en fonction du premier spectre (9) d'énergie et d'une troisième plage (EB3) d'énergie, et un quatrième signal de mesure en fonction du deuxième spectre (10) d'énergie et de la première plage (EB1) d'énergie ou en fonction du deuxième spectre (10) d'énergie et d'une quatrième plage (EB4) d'énergie, et dans lequel le troisième et le quatrième signal de mesure entrent en outre aussi dans le stade de la production (S5).

7. Procédé suivant la revendication 6, dans lequel, lors de la production (S4) de l'ensemble spectral de données d'image de tomodensitométrie assistée par ordinateur, les signaux de mesure créés sont entrés dans l'ensemble de données d'image en étant pondérés au moyen de facteurs de pondération optimisés, et dans lequel on pondère de manière plus haute le premier signal de mesure que le troisième signal de mesure et on pondère de manière plus haute le deuxième signal que le quatrième signal de mesure.

8. Procédé suivant la revendication 7, dans lequel, lors de la production (S4) de l'ensemble spectral de données d'image de tomodensitométrie assistée par ordinateur, les signaux de mesure créés entrent dans l'ensemble de données d'image en étant pondérés au moyen de facteurs de pondération optimisés, et dans lequel les facteurs de pondération sont optimisés au moins sur la base d'un critère de la liste suivante
• une valeur de bruit d'image de l'ensemble de données d'image de tomodensitométrie assistée par ordinateur,
• une valeur de contraste d'image de l'ensemble de données d'image de tomodensitométrie assistée par ordinateur,
• une valeur de contraste de matériau de l'ensemble de données d'image de tomodensitométrie assistée par ordinateur,
• une valeur d'artéfact de l'ensemble de données d'image de tomodensitométrie assistée par ordinateur.

9. Procédé suivant l'une des revendications précédentes, dans lequel, lors de l'émission (S2), on émet sélectivement en alternance, soit le premier spectre (9) d'énergie, soit le deuxième spectre (10) d'énergie.

10. Procédé suivant l'une des revendications 1 à 8, dans lequel l'unité (7) de détecteur a un détecteur (72) de rayons X comptant les photons ayant une pluralité d'éléments de détection, et dans lequel on expose un premier nombre partiel de la pluralité d'éléments de détection au moyen de l'unité (3) de source de rayons X au premier spectre (9) d'énergie, et on expose un deuxième nombre partiel de la pluralité au deuxième spectre (10) d'énergie, et dans lequel on crée le premier signal de mesure au moyen du premier nombre partiel de la pluralité d'éléments de détection et le deuxième signal de mesure au moyen du deuxième nombre partiel de la pluralité d'éléments de détection.

11. Procédé suivant l'une des revendications 1 à 8, dans lequel l'unité (7) de détection a un premier détecteur (73) de rayons X comptant les photons et un deuxième détecteur (74) de rayons X comptant les photons disposé par rapport à lui de manière décalée angulairement, et l'unité (3) de source de rayons X a respectivement en opposition, une première source (33) de rayons X, qui émet le premier spectre (9) d'énergie, et une deuxième source (34) de rayons X, qui émet le deuxième spectre (10) d'énergie, et dans lequel on crée le premier signal de mesure au moyen du premier détecteur (73) de rayons X et le deuxième signal de mesure au moyen du deuxième détecteur (74) de rayons X.

12. Installation de production d'un ensemble spectral de données d'image de tomodensitométrie assistée par ordinateur, comprenant
- une unité (3) de source de rayons X constituée pour émettre du rayonnement (4) X ayant un premier spectre (9) d'énergie et du rayonnement (4) X ayant un deuxième spectre (10) d'énergie différent du premier,
- une unité (7) de détection comportant au moins un détecteur (71, 72, 73, 74) de rayons X comptant les photons et constituée pour créer, sur la base du rayonnement (4) X détecté, des signaux de mesure, qui sont résolus au moins en une première plage (EB1) d'énergie adaptable et en deuxième plage (EB2) d'énergie adaptable,
- une unité (11) d'adaptation constituée pour adapter la première plage (EB1) d'énergie et la deuxième plage (EB2) d'énergie en fonction du premier spectre (9) d'énergie et du deuxième spectre (10) d'énergie, dans lequel respectivement au moins une énergie (GE1, GE2) limite d'une plage (EB1, EB2) d'énergie respective est adaptée,
- une unité (13) de traitement d'image constituée pour produire l'ensemble spectral de données d'image de tomodensitométrie assistée par ordinateur au moyen d'une technique spectrale de traitement d'image reposant sur les signaux de mesure créés, dans lequel au moins un premier signal de mesure, créé en fonction de la première plage (EB1) et du deuxième spectre (9) d'énergie, et un deuxième signal de mesure, créé en fonction de la deuxième (EB2) d'énergie et du deuxième spectre (10) d'énergie, entrent dans la production,
- une interface (15) d'émission de l'ensemble spectral de données d'image de tomodensitométrie assistée par ordinateur,
**caractérisée en ce que** l'installation comprend en outre
- une unité (25) d'optimisation, constituée pour la détermination (S0) d'une valeur d'énergie optimisée pour la au moins une énergie (GE1, GE2) limite respective de la première plage (EB1) et de la deuxième plage (EB2) d'énergie, au moins sur la base du premier spectre (9) d'énergie et du deuxième spectre (10) d'énergie, au moyen d'une unité (25) d'optimisation, dans lequel en outre on optimise au moins un critère de la liste suivante
• une valeur de bruit d'image de l'ensemble de données d'image de tomodensitométrie assistée par ordinateur,
• une valeur de contraste d'image de l'ensemble de données d'image de tomodensitométrie assistée par ordinateur,
• une valeur de contraste de matériau de l'ensemble de données d'image de tomodensitométrie assistée par ordinateur,
• une valeur d'artéfact de l'ensemble de données d'image de tomodensitométrie assistée par ordinateur,
• une valeur du chevauchement spectral entre le premier spectre (9) d'énergie et la deuxième plage (EB2) d'énergie et/ou entre le deuxième spectre (10) d'énergie et la première plage (EB1) d'énergie, et
dans laquelle l'adaptation (S1) de la première plage (EB1) d'énergie et de la deuxième plage (EB2) d'énergie repose sur la valeur d'énergie optimisée de la au moins une énergie (GE1, GE2) limite respective.

13. Installation suivant la revendication 12, constituée en outre pour effectuer un procédé suivant l'une des revendications 2 à 11.

14. Système (1) de tomodensitométrie assistée par ordinateur comprenant une installation suivant l'une des revendications 12 ou 13.
